(19) Europäisches Patentamt
European Patent Office
Office européen des brevets

(11) EP 4 778 467 A1

(12) **EUROPEAN PATENT APPLICATION**

(43) Date of publication:
**22.07.2026 Bulletin 2026/30**

(21) Application number: **26152182.7**

(22) Date of filing: **15.01.2026**

(51) International Patent Classification (IPC):
***A61B 6/06*** *(2006.01)* ***A61B 6/00*** *(2024.01)*
***A61B 6/58*** *(2024.01)* *A61B 6/04* *(2006.01)*
*A61B 6/42* *(2024.01)*

(52) Cooperative Patent Classification (CPC):
**A61B 6/06; A61B 6/542; A61B 6/545;**
A61B 6/0407; A61B 6/4283; A61B 6/4452;
A61B 6/587

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **16.01.2025 JP 2025006471**

(71) Applicant: **FUJIFILM Corporation**
**Tokyo 106-8620 (JP)**

(72) Inventors:
- **SAKAI, Yuji**
  **Tokyo, 106-8620 (JP)**
- **TAKEUCHI, Yumiko**
  **Tokyo, 106-8620 (JP)**
- **KITAMURA, Masataka**
  **Tokyo, 106-8620 (JP)**

(74) Representative: **Dehns Germany Partnerschaft mbB**
**Theresienstraße 6-8**
**80333 München (DE)**

# (54) X-RAY IMAGING APPARATUS AND AUTOMATIC CONTROL METHOD FOR X-RAY COLLIMATOR APERTURE

(57) Provided is a technique capable of automatically setting an irradiation range such that an effective image receiving surface of an FPD can be utilized to a maximum extent, regardless of a size of the FPD.

An X-ray imaging apparatus includes: an X-ray irradiation unit (10) including an X-ray tube (12) and an X-ray collimator (13); an imaging table (20) provided with an accommodating portion that accommodates an X-ray detector (40); and a processor (70) configured to control the X-ray irradiation unit (10), in which the processor (70) has an aperture automatic control function of detecting a size of the X-ray detector (40) and a distance between the X-ray tube (12) and the X-ray detector (40) and automatically adjusting an aperture of the X-ray collimator (13) in accordance with the size of the X-ray detector (40) based on a detection result.

FIG. 4

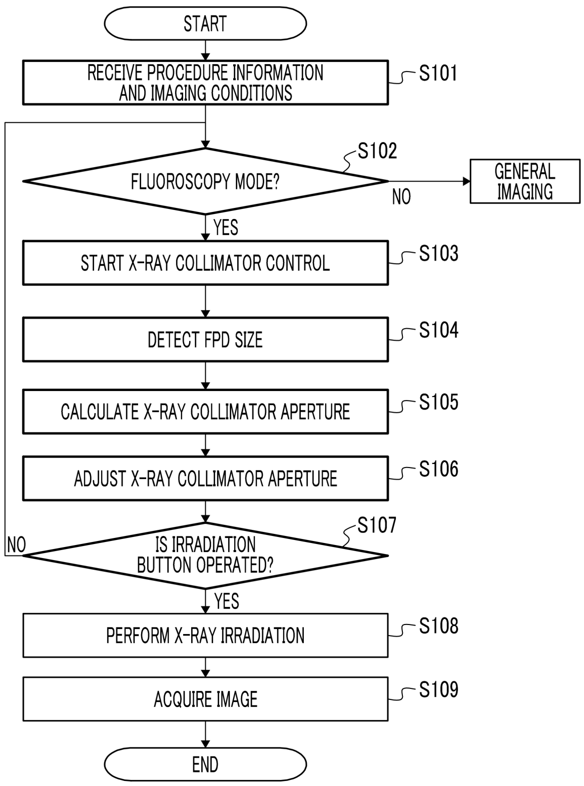


EP 4 778 467 A1

## Description

### CROSS-REFERENCE TO RELATED APPLICATIONS

The present application claims priority under 35 U.S.C. § 119 to Japanese Patent Application No. 2025-006471, filed January 16, 2025. Each of the above application(s) is hereby expressly incorporated by reference, in its entirety, into the present application.

### BACKGROUND OF THE INVENTION

1. Field of the Invention

The present invention relates to an X-ray imaging apparatus, and more particularly, to a control technique for an X-ray collimator.

2. Description of the Related Art

An X-ray imaging apparatus performs imaging with an X-ray detector disposed at a position facing an X-ray source that emits X-rays, with a subject interposed therebetween. The X-ray source is provided with an X-ray tube connected to a high-voltage generation device, an X-ray collimator that determines an irradiation range of X-rays emitted from the X-ray tube, and a collimator motor that opens and closes the X-ray collimator. The X-ray collimator is typically provided, for each of two orthogonal directions, for example, two directions such as an up-down direction and a left-right direction, with a pair of collimator blades that open and close, and drives the collimator motor to adjust a distance between the pair of blades (collimator aperture), thereby adjusting the irradiation range (irradiation field).

During imaging, an examination technologist or a physician (collectively referred to as a "user") operates operation buttons on an operation panel installed near the X-ray source or on a portable operation panel, and adjusts a distance (SID) between the X-ray source and the X-ray detector, the collimator aperture, and the like, thereby performing adjustment such that an X-ray irradiation range determined by the SID and the collimator aperture substantially coincides with the X-ray detector. In a case of oblique incidence imaging in which the subject is irradiated with X-rays from an oblique direction, an angle of the X-ray tube is also taken into consideration to adjust the X-ray irradiation range. Various types of X-ray detectors are present, but flat panel detectors (FPDs), which are versatile for both general imaging for acquiring still images (also simply referred to as "imaging") and fluoroscopy for acquiring moving images, are widely used.

In adjusting the X-ray irradiation field, for imaging in a fluoroscopy mode, it is defined by standards that the irradiation field of X-rays does not exceed the range of the X-ray detector, and the precision of adjustment of the aperture of the collimator blades is high. In addition, it is preferable that the entire image receiving surface of the X-ray detector (FPD) can be utilized as the irradiation field of X-rays, and it is desirable to secure the maximum possible irradiation field within constraints on the irradiation field.

Conventionally, since the collimator aperture has been adjusted by the user via the operation panel as described above, the adjustment is labor-intensive and imposes a heavy burden on the user. To address this problem, as a technique for automatically controlling an X-ray collimator, JP2005-31323A discloses providing means for adjusting a collimator by obtaining a collimation amount from dimensions of an irradiation field designated by a user on a radiation detection surface in a case where the user designates the dimensions of the irradiation field. Specifically, it is described that, in a case where the position and the size of a film serving as the X-ray detector are input, a collimator value (aperture) is calculated by using that information and a separately input sensor-to-tube distance (SID), and the irradiation field is controlled to achieve the calculated collimator value.

Additionally, JP2015-26313A further discloses a technique for controlling a collimator drive mechanism (collimator adjustment mechanism) such that radiation is emitted only within an incidence range of an FPD, based on information on an incidence range and a projection distance. Specifically, it is described that, in a case where an imaging mode is set, the incidence range and an imaging distance determined in advance according to imaging conditions are read out, a collimator aperture is calculated, and the collimator is controlled based thereon.

Further, JP2008-36314A discloses collimator adjustment in oblique incidence imaging, in which an irradiation direction of X-rays is inclined with respect to an imaging position of a subject for imaging, and a method for trimming the resulting image. However, JP2008-36314A does not mention automatic control of the collimator.

### SUMMARY OF THE INVENTION

The techniques described in JP2005-31323A and JP2015-26313A are techniques for controlling the collimator on the premise that the size of the film or the FPD is constant, and cannot accommodate the various FPDs currently in widespread use that have diverse sizes and effective image receiving surface sizes. In addition, in the technique described in JP2015-26313A, since the range of the irradiation field is determined based on the incidence range determined in advance, there is an issue that the user has to perform position adjustment of the FPD itself.

Further, there is also a possibility that, due to an erroneous operation, an FPD different from the FPD that should originally be used will be set on an imaging table, and in such a case, there is a risk that the irradiation field may exceed the size of the FPD; however, the conventional techniques cannot address such a situation.

An object of the present invention is to provide a technique capable of automatically setting an irradiation range such that an effective image receiving surface of an FPD can be utilized to a maximum extent, regardless of a size of the FPD.

In the present invention, in a state in which an X-ray detector is set in an accommodating portion, the size and the position of the X-ray detector are detected, an aperture of an X-ray collimator (an aperture of collimator blades) is calculated by using detected information, and an irradiation field corresponding to the FPD is automatically determined.

That is, according to an aspect of the present invention, there is provided an X-ray imaging apparatus comprising: an X-ray irradiation unit including an X-ray tube and an X-ray collimator; an imaging table provided with an accommodating portion that accommodates an X-ray detector; and a processor configured to control the X-ray irradiation unit, in which the processor has an aperture automatic control function of detecting a size of the X-ray detector and a distance between the X-ray tube and the X-ray detector and automatically adjusting an aperture of the X-ray collimator in accordance with the size of the X-ray detector based on a detection result.

In addition, according to another aspect of the present invention, there is provided an automatic control method for an X-ray collimator aperture of an X-ray imaging apparatus including an X-ray irradiation unit including an X-ray tube and an X-ray collimator, and an imaging table provided with an accommodating portion that accommodates an X-ray detector, the automatic control method comprising: a step of detecting a size of the X-ray detector accommodated in the accommodating portion and a distance between the X-ray tube and the X-ray detector; a step of calculating an X-ray collimator aperture corresponding to the size of the X-ray detector by using the detected size of the X-ray detector and the detected distance between the X-ray tube and the X-ray detector; and a step of automatically controlling the X-ray collimator to achieve the calculated X-ray collimator aperture.

According to the aspects of the present invention, in a state in which the X-ray detector is accommodated in the accommodating portion, by detecting a size and a position, or an orientation of the X-ray detector and by determining an irradiation range using detected information, it is possible to make adjustment by the user unnecessary and to enable the effective image receiving surface of the X-ray detector to be utilized to the maximum extent.

## BRIEF DESCRIPTION OF THE DRAWINGS

FIG. 1 is a diagram showing an overall outline of an X-ray imaging apparatus.

FIG. 2 is a block diagram of one embodiment of the X-ray imaging apparatus.

FIG. 3 is a diagram showing an example of an X-ray collimator.

FIG. 4 is a flowchart showing a flow of an operation of the X-ray imaging apparatus.

FIG. 5 is a diagram showing an example of a tray that accommodates an X-ray detector.

FIG. 6 is a diagram showing a display screen example of an operation panel.

FIG. 7 is a diagram illustrating a structure of the tray that accommodates an FPD and detection of an FPD size.

FIG. 8 is a diagram illustrating SIDs of decubitus imaging and upright imaging.

FIG. 9 is a diagram illustrating calculation of a collimator aperture of Embodiment 1.

FIGS. 10A to 10C are diagrams showing a relationship between an encoder indicating an amount of movement of the collimator, and the collimator aperture and an irradiation field.

FIG. 11 is a diagram showing an example of a table used for controlling an aperture of the X-ray collimator.

FIG. 12 is a flowchart of aperture control of the X-ray collimator of Embodiment 1.

FIGS. 13A to 13C are diagrams illustrating a modification example of Embodiment 1.

FIG. 14 is a diagram illustrating adjustment of an X-ray collimator of the modification example.

## DESCRIPTION OF THE PREFERRED EMBODIMENTS

Hereinafter, embodiments of an X-ray imaging apparatus according to the present invention will be described with reference to the drawings.

First, an overall outline of the X-ray imaging apparatus of the present embodiment will be described with reference to FIGS. 1 and 2.

As shown in FIG. 1, an X-ray imaging apparatus 1 comprises an X-ray irradiation unit 10 provided with an X-ray tube 12 and an X-ray collimator 13, an imaging table (20, 30) that supports a subject, and an X-ray tube drive mechanism unit 50 that moves the X-ray irradiation unit 10 (X-ray tube 12) in three orthogonal axial directions X, Y, and Z and in a rotation direction. The imaging table includes a decubitus imaging table 20 provided with a top plate 21 on which the subject is laid down, and an upright imaging stand 30 for performing upright imaging, and each is provided with an accommodating portion that accommodates an X-ray detector, which is typically an FPD 40, that detects X-rays emitted from the X-ray tube 12 and transmitted through the subject. In the following description, the X-ray detector will be referred to as an FPD 40.

In addition, the X-ray imaging apparatus 1 is provided with an operation unit 60 and a console 80 (also referred to as an operation console) for an operator to operate the X-ray irradiation unit 10 and the X-ray tube drive mechanism unit 50. The operation unit 60 is attached to the X-ray irradiation unit 10 and is movable independently of the X-ray tube 12 and the like. The console 80 is installed in an operation room that is shielded from an imaging room in which the X-ray irradiation unit 10 and the like are disposed.

Further, a processor 70 that controls imaging by controlling a high-voltage generation unit 15 (FIG. 2) connected to the X-ray tube, and that performs processing necessary for image generation by using information of transmitted X-rays detected by the FPD 40 and the like is connected to the X-ray imaging apparatus 1. The processor 70 may be accommodated in the console 80 or may be installed at a location separate from the X-ray imaging apparatus 1.

A configuration of the X-ray irradiation unit 10 is the same as that of a general X-ray irradiation unit and, as shown in FIG. 2, includes: the X-ray tube 12 that irradiates the subject with X-rays; the X-ray collimator 13 that sets an X-ray irradiation region with respect to the subject; an irradiation lamp 14 for confirming, with visible light, a region irradiated with X-rays by the X-ray collimator 13; and a support 11 that supports the X-ray tube 12 and the X-ray collimator 13 and the like.

As shown in FIG. 3, the X-ray collimator 13, in order to limit an irradiation field of X-rays emitted from the X-ray tube 12 in two axial directions (the X-axis direction and the Y-axis direction), is provided with a pair of collimator blades (X-axis collimator) 131 that open and close in the X-axis direction and a pair of collimator blades (Y-axis collimator) 132 that open and close in the Y-axis direction, and these are respectively driven by motors 133 and 134 and are configured such that amounts of driving thereof can be read by a potentiometer, encoders 135 and 136, or the like. In the following description, an encoder will be used representatively as a device that reads the amount of driving of the motor for description; however, known devices such as a potentiometer and an encoder can be used. Note that the pair of X-axis collimators 131 and the pair of Y-axis collimators 132 are each configured to open and close by symmetrically moving blades from a center portion of the collimator, but may be configured to independently move the pair of blades.

Values of the encoders are sent to the processor 70, whereby the processor 70 can obtain information on an aperture of the X-ray collimator. Additionally, in the present embodiment, the motor of the X-ray collimator is controlled by a control signal from the processor 70, and the aperture of the X-ray collimator is controlled. Control of the aperture of the X-ray collimator 13 by the processor 70 will be described in detail in the embodiment to be described below.

As shown in FIG. 1, the decubitus imaging table 20 comprises the top plate 21 and a support pedestal 22. In one embodiment, the support pedestal 22 has a structure that is extendable and retractable in the Z-axis direction, and a height of the top plate 21 can be adjusted. In addition, the top plate 21 is also movable in the X-axis direction and the Y-axis direction with respect to the support pedestal 22, and a position of the subject lying on the top plate 21 can be finely adjusted in the X-axis direction and the Y-axis direction with respect to the X-ray tube 12. A position of the top plate 21 is detected by a position sensor (not shown), and detected position information of the top plate 21 is sent to the processor.

A tray 25 (FIG. 2) that accommodates the FPD 40 is installed on the support pedestal 22 between the top plate 21 and the support pedestal 22. The tray 25 has an accommodating space matching a maximum-size FPD such that a plurality of types of FPDs can be accommodated. With respect to the accommodating space, for example, by inserting the FPD with a left end of the FPD along a left end of the accommodating space until a tip thereof abuts an end of the accommodating space, various FPDs are configured to be fixed at a set position. The tray 25 is provided with a sensor that discerns a type of the accommodated FPD, and a sense signal detected by the sensor is sent to the processor 70. The processor 70 can determine the type (size) of the FPD based on a signal from the sensor. The sensor may be configured using, for example, a plurality of photosensors; alternatively, a configuration in which information indicating a size or a type of the FPD can be labeled on a side surface or the like that does not affect an image receiving surface of the FPD 40, and the information can be read by attaching an optical reading device such as a camera or a barcode reader to a side surface of the tray 25, a mechanism that reads the size of the FPD 40 from a mechanical coupling state between the FPD 40 and the tray 25, or the like can also be used. Specific structures of the tray 25 and a sensor 41 will be described in detail in the embodiment to be described below.

Similarly, the upright imaging stand 30 is also provided with the accommodating portion (not shown) that accommodates the FPD 40, and the accommodating portion is provided with a sensor (not shown) that detects the size of the accommodated FPD 40, similarly to the tray 25 described above. A sense signal from the sensor installed in the accommodating portion of the upright imaging stand 30 is also sent to the processor 70.

The X-ray tube drive mechanism unit 50 includes a horizontal movement mechanism (an X-axis drive mechanism and a Y-axis drive mechanism) such as rails 55, a vertical movement mechanism (a Z-axis drive mechanism) (not shown), a rotation mechanism, and the like, and a part or all of the vertical movement mechanism and the rotation mechanism are incorporated into the X-ray irradiation unit 10. The X-ray tube drive mechanism unit 50 provided with such mechanisms can freely move and support the X-ray tube 12 in three axial directions, namely: a body-axis direction of the subject placed on the top plate 21, that is, a longitudinal direction of the top plate 21 (X-axis direction); a short-side direction of the top plate 21 orthogonal to the X-axis direction (Y-axis direction); and a vertical direction with respect to a surface of the top plate 21, which is orthogonal to the X-axis direction and the Y-axis direction (hereinafter, referred to as a Z-axis direction). Additionally, the X-ray tube 12 can be rotated with the Y-axis as an axis, for example, an irradiation direction of X-rays emitted from the X-ray tube 12 can be rotated at any angle (for example, within a range of $\pm 180$ degrees) between the vertical direction and the horizontal direction.

In addition, the X-ray tube drive mechanism unit 50 is provided with sensors 51 (an X-axis sensor, a Y-axis sensor, a Z-axis sensor, and a rotation sensor) that detect movement (an amount of movement) in each direction. Further, although not shown, the sensors 51 may include a sensor that detects a position of the support 11 of the X-ray tube 12. As the sensors 51, in addition to a mechanical sensor such as an encoder, known sensors such as an acceleration sensor and an optical or magnetic sensor can be employed. Sense signals of these sensors 51 are also sent to the processor 70.

As shown in FIG. 1, the operation unit 60 includes an operation handle 61, and by the operator's operation of the operation handle 61, movement and rotation of the X-ray irradiation unit 10 along each axis of the X-ray tube drive mechanism unit 50 can be performed. In addition to the operation handle 61, the operation unit 60 is provided with an operation panel 62 for allowing the operator to input information necessary for imaging and for presenting such information to the operator. Similarly to the operation unit 60, the console 80 is provided with various buttons (not shown) necessary for imaging, and a display device (a display panel 82) that displays a GUI and an X-ray image obtained by imaging. The operator inputs, via the console 80 (the display panel 82), procedure information such as whether imaging is performed in a decubitus position or in an upright position (information on an imaging body position), an imaging mode such as whether the imaging mode is general imaging (an imaging mode) or fluoroscopic imaging (a fluoroscopy mode) (information on a mode), imaging conditions, and the like. The imaging conditions include, for example, X-ray intensity, an X-ray irradiation rate, an irradiation field of view, and SID (a distance between the X-ray tube 12 and the FPD image receiving surface), and the like.

The operation panel 62 and the display panel 82 of the console 80 are complementary means for receiving inputs such as the operator's instructions, selections, and setting of values, and although inputs of the same items can be performed by both, the operation panel 62 installed in the imaging room is mainly used by the operator to confirm information input from the console 80, to perform adjustments after preparation for imaging, and to operate start of irradiation. In the following description, regarding operations via the operation panel 62 or the display panel 82 of the console 80, unless otherwise distinguished, the operation of the operation panel 62 will be described representatively, but the same operation performed via the display panel 82 is also included.

The processor 70 performs control of the X-ray imaging apparatus and computations/processing necessary for image generation, and, although the configuration thereof is not particularly limited, the processor 70 can be configured by one or more pieces of hardware or by a combination of hardware and programs. The types of hardware are not limited, and, for example, the processor can be configured by a central processing unit (CPU), a micro processing unit (MPU), a programmable logic device such as a field programmable gate array (FPGA), a dedicated circuit for executing specific processing such as an application specific integrated circuit (ASIC), or hardware such as a graphic processing unit (GPU) or a neural processing unit (NPU). Further, the types of hardware may be a combination of different types of hardware. In a case where a plurality of pieces of hardware are configured to execute one or more processes of a certain processor, the plurality of pieces of hardware may be present in devices physically separate from each other or may be present in the same device.

In a case where the processor 70 is implemented by a combination of hardware and programs, the programs may be software such as firmware or microcode. Further, the programs may be, for example, a group of program modules, and respective functions thereof may be implemented by processors configured to execute the respective functions. The programs may be a program code or a plurality of code segments stored in one or more non-transitory computer-readable media (for example, storage media, other storages, or the like).

In the embodiment shown in FIG. 2, as functions implemented by the processor 70, there are provided: an irradiation control unit 71 that controls a timing of X-ray irradiation from the X-ray tube 12 and the like; a collimator control unit 73 that controls the aperture of the X-ray collimator 13; a display control unit 74 that controls displays of the operation panel 62 and the display panel 82; and an image processing unit 72 that receives an X-ray transmission signal from the X-ray detector (FPD 40) and performs generation, storage, display, and the like of X-ray images. Note that the functions of the processor 70 are not limited to those shown in FIG. 2, and it is also possible to implement a part thereof with a processing device separate from the X-ray imaging apparatus 1, or to add other functions not shown in FIG. 2.

Next, an outline of the operation of the X-ray imaging apparatus having the above-described configuration will be described.

FIG. 4 shows a flow of an operation in a case of imaging in the fluoroscopy mode.

First, after the operator performs procedures such as: inserting the FPD 40 into the tray 25 and setting the FPD 40 on the decubitus imaging table 20; laying a subject 100 on the top plate 21 such that an imaging site of the subject 100 is located on the FPD 40; and inputting, via the console 80, the procedure information such as whether imaging is performed in a decubitus position or in an upright position, and imaging conditions such as a tube voltage, a tube current, an imaging distance (SID), and a range of the irradiation field (S101), the operator operates the X-ray tube drive mechanism unit 50 to position the X-ray tube 12 at a predetermined position. The predetermined position is a position at which, typically, the irradiation direction of X-rays is the vertical direction and an irradiation center coincides with a center (a predetermined range) of the FPD 40. The position of the FPD 40 is detected as a center position by, in advance, registering center position coordinates and by determining coincidence with position coordinates acquired from the drive mechanism units in X-axis, Y-axis, and Z-axis directions. Note that, in the present embodiment, the FPD 40 is assumed to be fixed to the decubitus imaging table 20.

After preparation for imaging is completed, in a case where the fluoroscopy mode is set (S102), the processor 70 starts control of the X-ray collimator aperture (S103). Control of the X-ray collimator aperture may allow selection between control for automatically obtaining a maximum irradiation field and a manual control mode in which the aperture follows manual operation. In a case where automatic control of the collimator aperture is started (S103), the collimator control unit 73 reads the orientation and the size of the FPD 40 accommodated in the tray 25 based on sense signals from sensors of the tray 25 (S104). Additionally, the collimator control unit 73 acquires the position of the X-ray tube 12 based on sense signals from the respective sensors 51 of the X-ray tube drive mechanism unit 50, calculates a position (SID) from the X-ray tube 12 to the image receiving surface of the FPD 40, and calculates the aperture of the X-ray collimator by using the FPD size and the SID (S105). In control for obtaining the maximum irradiation field, an aperture is calculated such that the irradiation field of X-rays coincides with the image receiving surface (a maximum area) of the FPD.

Subsequently, the collimator control unit 73 controls the motor of each axis of the X-ray collimator to achieve the calculated aperture of the X-ray collimator 13 (S106). After the X-ray collimator 13 has been adjusted to be the same as the size of the FPD image receiving surface such that the X-ray collimator 13 is the same as the size of the FPD image receiving surface, in a case where a technologist, a physician, or the like (hereinafter, referred to as an "operator") operates an irradiation button of the operation panel 62 (S107), the irradiation control unit 71 receives the operation, controls the high-voltage generation unit 15, and performs X-ray irradiation toward the FPD 40 (S108). The image processing unit 72 receives a signal corresponding to transmitted X-rays from the FPD 40 and performs generation, storage, and the like of images (S109). In a case where automatic collimator control is not selected, the manually adjusted collimator aperture is maintained, and in a case where the irradiation button is operated (S107), irradiation (S108) and image acquisition (S109) are performed. Note that, at a point in time when automatic adjustment is completed, the display control unit 74 may perform a display on the operation panel 62 indicating that adjustment of the collimator has been completed and that irradiation is permitted. In such a case, the operator confirms the display and operates the irradiation button.

According to the present embodiment, in the fluoroscopy mode, in a case where automatic control of the X-ray collimator 13 is performed, the processor 70 reads the size of the FPD 40 based on sense signals from the tray 25 (accommodating portion) that accommodates the FPD 40, and, by using size information and SID information, calculates the irradiation field of view in which the image receiving surface of the FPD 40 is utilized to a maximum extent. The processor 70 automatically controls the aperture of the X-ray collimator to achieve the calculated irradiation field of view.

As a result, the operator can, for various sizes of FPDs, reduce the effort of collimator aperture adjustment while utilizing the image receiving surfaces thereof to the maximum extent.

Based on the above embodiment, specific embodiments of control of the X-ray collimator aperture will be further described. In the following embodiments, with respect to configurations overlapping those of the above-described embodiment, FIGS. 1 to 3 are used, and the description is omitted or simplified.

Embodiment 1

Control of the X-ray collimator of the present embodiment is characterized in that a configuration is provided in which an automatic control mode, in which the X-ray collimator is automatically controlled, and a manual control mode, in which the operator manually adjusts the irradiation field in accordance with the irradiation field size, can be set (Feature 1).

The automatic control mode is a control in which the processor 70 automatically detects the size (including the orientation) of the FPD and adjusts the aperture of the X-ray collimator in accordance with the maximum area of the image receiving surface of the FPD. In the automatic control mode, for example, even in a case where the SID is changed by vertical movement or the like of the imaging table during automatic control, the collimator aperture is automatically adjusted such that the irradiation field is enlarged or reduced in accordance with the FPD image receiving surface.

In the manual control mode, the operator achieves a desired irradiation field by manually adjusting the collimator aperture, or by operating vertical movement, lateral movement, or the like of the imaging table with a fixed collimator aperture to adjust the SID and the like. In the manual control mode, in a case where the collimator aperture that follows the desired irradiation field can no longer be achieved because of movement of the imaging table and the like, the processor 70 performs control to disable X-ray irradiation.

Further, collimator control of the present embodiment is characterized in that information for the operator to confirm a situation during collimator control and information related to permission or prohibition of irradiation are presented via the operation panel (Feature 2).

Hereinafter, specific configurations for achieving these features will be described.

Details of control units implemented by the processor 70 related to collimator aperture control are shown in FIG. 5.

As illustrated, in addition to the irradiation control unit 71, the collimator control unit 73, and the display control unit 74 shown in FIG. 2, the processor 70 is provided with: a collimator aperture calculation unit 75; an FPD detection unit 76; an SID calculation unit 77; a body position determination unit 78 that determines an imaging body position based on information (procedure information and the like) input from the console 80 as to whether imaging is decubitus imaging or upright imaging; and a mode determination unit 79 that determines whether the console 80 is in the imaging mode or the fluoroscopy mode. Functions of respective units of the processor 70 will be described in detail below.

In a case where the automatic control mode is set as a mode of collimator aperture control described above, the collimator control unit 73 acquires necessary information from the collimator aperture calculation unit 75, the FPD detection unit 76, the SID calculation unit 77, the body position determination unit 78, and the mode determination unit 79, performs control of the automatic control mode, and, furthermore, the collimator control unit 73 compares the size of the FPD detected by the FPD detection unit 76 with the irradiation field determined by the collimator aperture calculated by the collimator aperture calculation unit 75, and passes information such as permission or prohibition of irradiation to the display control unit 74. Details of the collimator control unit 73 and of the respective units related to collimator control will be described below.

The display control unit 74 mainly performs control of displays related to the collimator aperture (collimator aperture display control unit), detection of button operations of the operation panel 62 (a button operation detection unit), and display of operation buttons (a button display control unit).

The collimator aperture display control unit performs display control of information to be presented to the operator, such as a display indicating that the automatic control mode is set and a display issuing notification of permission or prohibition of irradiation. The button operation detection unit detects button operations by the operator and passes processing corresponding to an operated button to the processor (the collimator control unit 73). The button display control unit performs processing such as changing a display aspect of the button, for example, a color or brightness of the button, a color of a frame of the button, or the like, in a case where the button is operated and corresponding processing is performed.

FIG. 6 shows an example of a display screen of the operation panel 62 controlled by the display control unit 74. As illustrated, the operation panel is provided with a plurality of buttons (GUI), such as mode selection buttons 621 and 622 for selecting either the imaging mode or the fluoroscopy mode, body position selection buttons 623 and 624 for selecting a decubitus position or an upright position, start buttons 625 and 626 for issuing instructions to start fluoroscopy or imaging, and a button 627 (a "MAX" button in the drawing) for selecting automatic setting of the X-ray collimator, and a display region 628 for displaying procedure information and conditions input from the console 80. In the illustrated example, the size of the FPD and the irradiation field size are displayed in the display region 628.

As described above, in control of the X-ray collimator of the present embodiment, a configuration is provided in which the automatic control mode, in which the X-ray collimator is automatically controlled in accordance with the maximum area of the image receiving surface of the FPD, and the manual control mode, in which the operator manually performs adjustment in accordance with the irradiation field size, can be set, and in the example of the operation panel shown in FIG. 6, in a case where the operator operates the "MAX" button 627, the automatic control mode is turned "ON", and automatic control to be described below starts. Additionally, in a case where the "MAX" button 627 is operated again, the automatic control mode is turned "OFF", and an aperture at that time is maintained. A configuration may also be employed in which another button for canceling the automatic control mode is provided.

The display control unit 74 detects operations of respective buttons by the operator and sends detected results to the processor 70. For example, operation results of the mode selection buttons 621 and 622 for selecting either the imaging mode or the fluoroscopy mode are sent to the mode determination unit 79, operation results of the body position selection buttons 623 and 624 are sent to the body position determination unit 78, and an operation result of the "MAX" button 627 is sent to the collimator aperture calculation unit 75. In addition, in a case where the button operation of the "MAX" button 627 is performed, the display control unit 74 displays on the operation panel 62 that the automatic control function of the collimator aperture has been turned ON. A manner of display is not particularly limited; however, for example, a color of a frame of an operated button or a brightness or a color of the button itself may be made different. For other buttons as well, a configuration may be employed in which display of operation buttons is controlled such that which button has been operated and which processing is in an "ON" state can be recognized.

The collimator control unit 73 includes a collimator aperture determination unit 731 that inputs sense signals from an X-axis collimator sensor and a Y-axis collimator sensor, and that determines, for each of the X-axis collimator and the Y-axis collimator, the collimator aperture. In the collimator control unit 73, information on the collimator aperture determined by the collimator aperture determination unit 731 is sent to the display control unit 74 and is displayed on the operation panel 62, for example, in the display region 628.

Further, the collimator control unit 73 determines whether or not irradiation is permitted, based on the collimator aperture determined by the collimator aperture determination unit 731, sends the determination result to the display control unit 74, and displays permission or prohibition of irradiation on the operation panel 62. Determination of whether or not irradiation is permitted is made by comparing the size of the FPD 40 with the irradiation field determined by the collimator aperture calculated by the collimator aperture calculation unit 75, and in a case where FPD size > irradiation field size or FPD size = irradiation field size, irradiation is permitted, and in a case where FPD size < irradiation field size, irradiation is prohibited. A manner of display of permission or prohibition of irradiation is not particularly limited; however, for example, the "MAX" button 627 is activated in a case where irradiation is permitted, and is inactivated in a case where irradiation is prohibited, a color of a frame surrounding the "MAX" button 627 is changed to a warning color (for example, gray), or permission or prohibition of irradiation is displayed as character information in the display region 628 or the like.

The FPD detection unit 76 includes a detection unit that detects the FPD of the decubitus imaging table 20 and a detection unit that detects the FPD of the upright imaging stand 30, inputs sense signals from FPD detection sensors respectively provided in the decubitus imaging table 20 and the upright imaging stand 30, and detects whether or not the FPD 40 is disposed at an appropriate position of the decubitus imaging table 20 or the upright imaging stand 30.

As an example of FPD detection, a configuration of the tray 25 installed in the decubitus imaging table 20, and the size and the orientation of the FPD 40 will be described. Here, for simplicity of description, a case will be described in which, as the FPD 40, a size A (17 inches $\times$ 17 inches) and a size B (17 inches $\times$ 14 inches) are used.

As shown in a part (a) of FIG. 7, the tray 25 has a substantially rectangular outer shape, and a plurality of (three in total in the drawing) photosensors (Sensor 1 to Sensor 3) are provided along two adjacent sides of the rectangle. These sensors are disposed such that an interval between Sensor 1 and Sensor 2 and an interval between Sensor 2 and Sensor 3 are each greater than 14 inches and less than 17 inches. As a result, as shown in a part (b) of FIG. 7, in a case where the FPD 40 of the size A is inserted, all three sensors are covered by the FPD 40, and sense signals are issued from all three sensors. In addition, as shown in a part (c) of FIG. 7, in a case where the FPD 40 of the size B is inserted in a lateral orientation (landscape), only Sensor 1 is covered by the FPD 40, and a sense signal is issued from this sensor. In a case where the FPD 40 of the size B is inserted in an upright orientation (portrait) with the FPD 40 aligned to the left of the tray 25 (a part (d) of FIG. 7), two sensors disposed along the left side, that is, Sensors 1 and 2, are covered by the FPD 40, and sense signals are outputted from the two sensors.

Based on the number of sense signals received, the collimator control unit 73 can discern four states ((a) to (d)) including presence or absence of the FPD as shown in the table below FIG. 7, and can determine which size of the FPD is disposed in which orientation on the tray 25. A guide can be provided in the tray 25 such that FPDs having different aspect ratios (for example, the FPD of the size B) are inserted with alignment to the left side of the tray 25, whereby the size and the orientation can be reliably detected.

FIG. 7 shows a configuration for discerning two sizes of FPDs; however, by increasing the number of sensors and appropriately setting sensor intervals based on the size of the FPD 40, a plurality of sizes can be discerned. In addition, although the disposition of sensors in FIG. 7 is a disposition for detecting the size in a case where the FPD 40 is disposed with alignment to the left end (or the right end) of the tray 25, it is also possible to employ a disposition of sensors capable of detecting the size in a case where the FPD 40 is disposed at the center of the tray 25.

The SID calculation unit 77 calculates the SID by using the procedure information input to the body position determination unit 78 and the position of the X-ray tube 12 detected by the sensors 51 (including a sensor that detects the position of the support 11 of the X-ray tube 12) of the drive mechanism unit 50 and the position of the set FPD. The procedure information includes information as to whether imaging is the decubitus imaging or the upright imaging, and the SID calculation unit 77 calculates, as the SID, in a case of decubitus imaging as shown on the upper side of FIG. 8, a distance between the FPD image receiving surface installed in the decubitus imaging table and the X-ray tube 12, and, in a case of upright imaging as shown on the lower side of FIG. 8, a distance between the FPD image receiving surface installed in the upright imaging stand and the X-ray tube 12.

The collimator aperture calculation unit 75, based on the SID calculated by the SID calculation unit 77, the procedure information and fluoroscopy mode information input via the operation panel 62 or the console 80, and the information input by button operations of the operation panel, calculates a target value of the collimator aperture, for example, a collimator angle by which the irradiation field that substantially coincides with the size of the FPD image receiving surface is obtained, and performs calculation of the collimator aperture for each of the X-axis collimator and the Y-axis collimator (an X-axis collimator aperture calculation unit and a Y-axis collimator aperture calculation unit).

A specific method of calculating the collimator aperture will be described with reference to FIG. 9. In the present embodiment, the collimator aperture is normalized, and by adjusting the normalized value in accordance with actual SID (the distance between the X-ray tube 12 and the FPD image receiving surface), the collimator aperture by which the irradiation field coincides with a maximum width of the FPD image receiving surface is calculated. In FIG. 9, for simplicity of description, a case is shown in which the collimator aperture of one of the X-axis and the Y-axis is calculated.

As shown on the left side of FIG. 9, the collimator aperture in a case where the collimator is fully open is defined as amax, and the irradiation field size in a case where the SID is set as 1 m is defined as Amax. Since amax is a fixed value determined by the X-ray collimator, Amax is also a predetermined value. A ratio between Amax and amax is defined as a normalized value, and, in a case where an actually set value of the SID is defined as D and the size of the image receiving surface of the FPD 40 to be used is defined as W, a collimator aperture a that achieves the irradiation field matching the maximum width of the image receiving surface is calculated by the following Equation (1).

$$a = [amax/Amax] \times [W/D] \quad (1)$$

That is, the collimator aperture calculation unit 75 uses D (the value of the SID) calculated by the SID calculation unit 77 and the size W of the image receiving surface determined by the size of the FPD 40 detected by the FPD detection unit 76 to calculate the collimator aperture a, and sends the collimator aperture a to the collimator control unit 73.

The collimator control unit 73 controls the X-axis collimator and the Y-axis collimator by using the value of the collimator aperture calculated by the collimator aperture calculation unit 75, and controls the collimator aperture to achieve a predetermined irradiation field size. In control of the collimator aperture, current positions of the X-axis collimator and the Y-axis collimator are read from the encoders and the like, and the motors 133 and 134 of the X-axis collimator 131 and the Y-axis collimator 132 are controlled such that the X-axis collimator and the Y-axis collimator move to positions at which the collimator aperture is the calculated aperture. This control can be achieved by preparing in advance a table of a relationship between read values of the encoders 135 and 136 and the collimator aperture, and a relationship between the collimator aperture for each SID and the irradiation field size, and by determining, from the table, a position (a read value) of the collimator corresponding to the SID.

In a case where the collimator aperture changes from fully closed to fully open, the relationship between the encoder and the collimator aperture is a relationship shown in FIG. 10A, and in a case where this is expressed as a relationship with the irradiation field Amax at SID = 1 m, the relationship is shown by the graph in FIG. 10B. In a case where the SID is greater than 1 m, the irradiation field widens, and, for example, at SID = 1.4 m, it changes as shown in FIG. 10C. FIG. 11 shows an example of a table used for collimator control, which is created based on such relationships. The illustrated example is an example in which, for three SIDs (SID = 1 m, 1.5 m, and 2.0 m), the relationship between the size of the irradiation field of view and the collimator aperture and the relationship between the collimator aperture and the encoder read values are defined. Here again, for simplicity of description, only a collimator in one axial direction is shown, but in the two axial directions X and Y, such tables are respectively prepared.

From the table of FIG. 11, the collimator aperture is calculated by linear interpolation using two adjustment points. Specifically, by using the value on the image receiving surface at the position of SID = 1 m in a case where the collimator aperture is fully closed (Adjustment Point 1) and the value in a case where the collimator aperture is fully open (Adjustment Point 2), linear interpolation is performed to determine the collimator aperture that yields the maximum irradiation size W determined by the FPD size. For example, in a case where the aperture on the image receiving surface at the position of SID = 1 m corresponds to 20 cm, the aperture on the image receiving surface at the position of SID = 2 m is 40 cm; therefore, in order to keep the aperture at 20 cm on the image receiving surface even at SID = 2 m, the collimator aperture is set to 1/2.

By using such a table, simple control is possible. In the above description, the collimator aperture has been calculated from the table values according to the FPD size by linear interpolation instead of using the discrete values of the table; however, values for each SID (or coefficients with reference to SID = 1 m) may be determined in advance.

Next, an example of the flow of X-ray collimator control of the X-ray imaging apparatus of the present embodiment, based on the above-described configuration, will be described with reference to FIG. 12. Here, as an example, a case will be described in which imaging is performed by using the decubitus imaging table.

First, prior to imaging, after the procedure information, the imaging conditions, and the like are input via the console 80 (FIG. 4, S101), the imaging site of the subject is positioned at the imaging position, the X-ray tube 12 is moved to the imaging site, a state in which the center of the FPD set on the imaging table and the X-ray tube 12 face each other is confirmed, and control of the fluoroscopy mode is started (S111).

Control of the fluoroscopy mode is started after completion of preparation for imaging by the processor 70 reading mode information input from the console 80 or the operation panel 62 and the mode determination unit 79 determining that the mode is the fluoroscopy mode. Subsequently, the processor 70 starts the X-ray collimator control function (control of the automatic control mode) in a case where the "MAX" button 627 of the operation panel 62 is operated (S200). In a case where the "MAX" button 627 is not operated, the automatic control mode is not entered, and the operator performs manual adjustment of the irradiation field.

In a case where control of the automatic control mode (S200) is started, the body position determination unit 78 reads information related to the body position from the procedure information input, for example, via the console 80, and determines whether imaging is performed in a decubitus position or an upright position (S113). The processor 70 performs calculation of SID and detection of the FPD according to the determination result.

As shown in FIG. 8, in a case of decubitus imaging, the FPD detection unit 76 receives a sense signal from the FPD sensor 41 of the decubitus imaging table 20 and determines presence or absence of the FPD and the size and the orientation of the FPD (S114). In addition, in a case of upright imaging, the FPD detection unit 76 determines the size of the FPD and the like by using a sense signal from the FPD sensor of the upright imaging stand. As described with reference to FIG. 7, the determination of the size and the like is performed from the number and disposition of photosensors that have issued sense signals, among the plurality of photosensors. The size of the FPD determined by the FPD sensor is displayed by the display control unit 74 in the display region 628 of the operation panel. In a case where the FPD 40 is not set at a predetermined position, an alert may be issued, such as displaying that fact on the operation panel 62.

The SID calculation unit 77 acquires the position information of the X-ray tube 12 from the sensors 51 for position detection of the X-ray tube 12, and, by using information as to which imaging table (the decubitus imaging table or the upright imaging stand) is used from the procedure information (information on the body position), calculates the distance SID from the center of the X-ray tube 12 to the center of the FPD image receiving surface set on the imaging table (S115). Subsequently, the collimator aperture calculation unit 75 calculates, by Equation (1) described above, apertures of the X-axis collimator and the Y-axis collimator corresponding to the size of the FPD by using the size of the FPD (the size in the X-axis direction and the size in the Y-axis direction) detected by the FPD detection unit 76 and the SID calculated by the SID calculation unit 77 (S116). The collimator control unit 73 determines, from the table, the amounts of movement (encoder values) of the X-axis collimator and the Y-axis collimator by using the collimator apertures calculated by the collimator aperture calculation unit 75 and the current positions of the collimators determined by the collimator aperture determination unit 731, and controls the collimator drive motors such that the X-axis collimator and the Y-axis collimator move by the amounts of movement (S117).

In a case where the irradiation field is determined by control of the collimator aperture, the processor 70 (the collimator control unit 73) sends that information to the display control unit 74 and displays the information on the operation panel 62 (in the display region 628). Further, it is determined whether or not the calculated irradiation field exceeds the detected size of the FPD 40, and, in a case where the size of the irradiation field is equal to or less than the size of the FPD 40, the display control unit 74 displays on the operation panel 62 that irradiation is permitted. For example, an irradiation button (fluoroscopy start button 626), which has been inactive until then, is activated.

In a case where the operator confirms the display of the operation panel 62, or operates the irradiation button by activation of the irradiation button, the irradiation control unit 71 irradiates the FPD 40 with X-rays (S118). In imaging in the fluoroscopy mode, while monitoring that a positional relationship between the X-ray tube 12 and the FPD 40 has not changed, X-ray irradiation and acquisition of fluoroscopic images are performed for a predetermined time (S119). While the fluoroscopy mode continues, while constantly monitoring a change in the distance SID between the X-ray tube 12 and the FPD (image receiving surface), automatic control is continued. That is, processing of steps S113 to S117 is repeatedly performed.

In a case where there is no operation of the "MAX" button in the fluoroscopy mode, the aperture of the X-ray collimator is maintained at the aperture at the time of initial setting or manual setting, and fluoroscopy by operation of the fluoroscopy start button is performed (the manual control mode of the collimator aperture). Although not shown in FIG. 12, in the manual control mode as well, the irradiation field is calculated from the set collimator aperture and the calculated SID, a step of confirming that the calculated irradiation field does not exceed the FPD size is executed, and, for example, by deactivating the irradiation button, X-ray irradiation exceeding the FPD size is prevented.

As described above, according to the present embodiment, the FPD detection unit detects the size and the orientation of the FPD, and it is possible to ensure that the X-ray irradiation range does not protrude beyond the image receiving surface while utilizing the size of the image receiving surface of the actually disposed FPD to the maximum extent. In addition, since control of the collimator aperture is performed under the control of the processor, it is possible to significantly reduce the operator's effort and time.

Further, according to the present embodiment, the operator can always understand, via the operation panel, a status of automatic control, erroneous operation can be prevented, and fluoroscopy can be performed safely while securing conditions necessary for fluoroscopy.

Although the flow of control of the present embodiment has been described above by using decubitus imaging as an example, in a case of upright imaging, except that detection of the FPD uses a sense signal from the FPD sensor of the upright imaging stand and that the SID calculation unit 77 calculates SID with reference to the upright imaging stand, the control of the X-ray collimator can be performed in the same manner as in the case of decubitus imaging. Additionally, the configuration of the operation panel and the configuration of the FPD detection unit (tray) exemplified in the present embodiment can be variously modified.

In addition, in the present embodiment described above, the automatic control mode and the manual control mode are switched by operation of the MAX button (Feature 1); however, it is also possible to set the automatic control mode in conjunction with the start of fluoroscopy, and such embodiments are also encompassed in the present invention. Further, in addition to the configuration for switching between the automatic control mode and the manual control mode in the present embodiment, numerical values, determination results, and the like obtained during collimator control are reflected in the display of the operation panel (Feature 2); however, in the present invention, it is not essential to include both Feature 1 and Feature 2, and an X-ray imaging apparatus provided with only one of them is also encompassed in the present invention.

Furthermore, in the present invention, the specific configurations shown in the present embodiment, for example, a configuration in which the tray 25 that accommodates the FPD 40 is provided with photosensors that detect the size and the orientation (accommodation direction) of the FPD 40; a configuration in which, for the collimator aperture that yields the maximum image receiving surface of the FPD 40, a correspondence relationship (table) with the SID is set in advance and aperture automatic control is performed based on this table; and a configuration in which, according to permission or prohibition of irradiation, the irradiation button is activated/deactivated, can each be variously modified. Additionally, it is not essential to include all of these configurations or their modification examples, and there may be embodiments of an X-ray imaging apparatus provided with any one or any two of them.

Modification Example

Next, as a modification example of the present embodiment, collimator control in a case where the positional relationship between the X-ray tube 12 and the FPD 40 has changed will be described.

In Embodiment 1, as shown in FIG. 13A, collimator aperture control is performed on the premise that the irradiation direction of X-rays emitted from the X-ray tube coincides with a line connecting the focal spot of the X-ray tube and the center position of the FPD, that is, that the irradiation direction of X-rays is vertical, and the center of the X-ray tube 12 coincides with the center of the FPD image receiving surface in the vertical direction. In order to confirm this premise, a configuration may also be employed in which, for example, before step S115 in FIG. 12, the processor executes a step of determining whether or not the line connecting the focal spot of the X-ray tube 12 and the center position of the X-ray detector coincides with the irradiation direction of X-rays emitted from the X-ray tube 12, and the automatic control function of the collimator aperture is executed only in a case where the line coincides with the irradiation direction.

In addition, in a case where a mechanism capable of independently adjusting the pair of X-ray collimators is provided, instead of adding the above-described determination step, it is also possible to perform control of the collimator aperture corresponding to a case where the irradiation direction of X-rays emitted from the X-ray tube 12 does not coincide with the line connecting the focal spot of the X-ray tube 12 and the center position of the FPD 40. In this case, the pair of collimator blades are individually moved to adjust control of the collimator aperture. For example, in a case where the positional relationship between the X-ray tube 12 and the FPD 40 is such that, as shown in FIG. 13B, one of the X-ray tube 12 and the FPD 40 is displaced in the X-axis direction or the Y-axis direction and the line connecting their center positions is inclined from the vertical direction, or, as shown in FIG. 13C, in oblique incidence imaging in which X-rays from the X-ray tube 12 are emitted to the subject from an oblique direction for imaging and the irradiation direction of X-rays is inclined with respect to the vertical direction, the pair of collimator blades are individually controlled.

For example, adjustment in a case of FIG. 13B is performed as follows.

First, an amount of displacement $\Delta x$ in a parallel direction between the X-ray tube 12 and the FPD 40 is calculated. The amount of displacement can be calculated, for example, by acquiring the position of the X-ray tube 12 (a position in a coordinate system of the apparatus) from sense signals from the sensors 51 that detect the position, with reference to the center position of the top plate 21 in the coordinate system of the apparatus and the FPD 40 accommodated therein. The center position of the FPD 40 can be calculated from the size of the FPD 40 detected by the FPD detection unit 76 and the relationship with the tray 25 in which the FPD 40 is accommodated. An amount of displacement ΔW of the irradiation field is equal to this amount of displacement Δx.

In a case where the X-axis collimator is taken as an example, the collimator aperture calculation unit 75 first calculates the aperture a of the X-axis collimator in the same manner as in Embodiment 1. Subsequently, the aperture (a/2) of the left and right X-axis collimator blades is increased/decreased by an aperture Δa corresponding to the amount of displacement Δx in the X-axis direction (the amount of displacement ΔW of the irradiation field). That is, for one collimator blade, the aperture Δa corresponding to the amount of displacement ΔW is subtracted from 1/2 of the aperture a of the X-ray collimator calculated by the collimator aperture calculation unit 75, and, for the other collimator blade, the aperture Δa corresponding to the amount of displacement ΔW is added to 1/2 of the aperture a of the X-ray collimator calculated by the collimator aperture calculation unit 75. The collimator aperture Δa corresponding to the amount of displacement ΔW can be calculated based on a geometric relationship between the irradiation field W obtained from the aperture a of the X-axis collimator and the irradiation field W + ΔW obtained from the aperture a/2 + Δa. In the geometric relationship, as shown in FIG. 14, since a ratio between the irradiation field W/2 and the aperture a/2 is the same as a ratio between the irradiation field (W/2 + ΔW) and (a/2 + Δa), Δa can be obtained from that relationship.

In a case of oblique incidence imaging shown in FIG. 13C, the irradiation field W determined by the collimator aperture calculated based on the size of the FPD 40 (the irradiation field that utilizes the image receiving surface to the maximum extent) widens as the irradiation angle is inclined. The collimator aperture calculation unit 75 adjusts the aperture such that the irradiation field is (W - ΔW) obtained by subtracting an error ΔW, which occurs due to inclination of the irradiation angle, from W. In this case as well, ΔW can be calculated from the geometric relationship between SID and the size of the FPD image receiving surface and from the oblique incidence angle. For each oblique incidence angle, an amount of adjustment of the irradiation field may be calculated in advance, and the value calculated in advance may be set as an adjustment value at oblique incidence.

As described above, according to the present modification example, even in a case where the X-ray tube 12 and the FPD 40 do not face each other, automatic control of the collimator aperture matching the size of the FPD 40 is possible. As a result, with respect to the positional relationship between the X-ray tube 12 and the FPD 40, a tolerance for alignment accuracy by the operator can be relaxed, and work effort can be reduced.

Further, by combining the technique disclosed in JP2008-36314A, in which the rotation angle of collimator blades is controlled at oblique incidence to perform proper trimming on an image, with the method of the present embodiment (modification example), it is possible to control the collimator aperture such that the X-ray irradiation field of view is equal to the size of the FPD image receiving surface, according to SID and the FPD.

Explanation of References

- 10: X-ray irradiation unit
- 20: decubitus imaging table
- 30: upright imaging stand
- 40: FPD (X-ray detector)
- 50: X-ray tube drive mechanism unit
- 60: operation unit
- 62: operation panel
- 70: processor
- 71: irradiation control unit
- 72: image processing unit
- 73: collimator control unit
- 74: display control unit
- 75: collimator aperture calculation unit
- 76: FPD detection unit
- 77: SID calculation unit
- 78: body position determination unit
- 79: mode determination unit
- 80: console

## Claims

**1.** An X-ray imaging apparatus comprising:

an X-ray irradiation unit including an X-ray tube and an X-ray collimator;
an imaging table provided with an accommodating portion that accommodates an X-ray detector; and
a processor configured to control the X-ray irradiation unit,
wherein the processor has an aperture automatic control function of detecting a size of the X-ray detector and a distance between the X-ray tube and the X-ray detector and automatically adjusting an aperture of the X-ray collimator in accordance with the size of the X-ray detector based on a detection result.

**2.** The X-ray imaging apparatus according to claim 1, wherein the processor is configured to:

control the X-ray irradiation unit according to any one of a fluoroscopy mode or an imaging mode; and
execute the aperture automatic control function only in imaging in the fluoroscopy mode.

3. The X-ray imaging apparatus according to claim 1,

wherein the accommodating portion includes a sensor that detects the X-ray detector, and
the processor is configured to discern the size of the X-ray detector by using a signal from the sensor.

4. The X-ray imaging apparatus according to claim 3,

wherein the sensor includes a plurality of photosensors disposed along a plane direction of the X-ray detector, and
the processor is configured to discern the size of the X-ray detector by using outputs of the plurality of photosensors.

5. The X-ray imaging apparatus according to claim 3, further comprising:

a drive mechanism unit configured to make the X-ray irradiation unit movable in each of an X-axis direction, a Y-axis direction, and a Z-axis direction, which are orthogonal to each other,
wherein the processor is configured to detect the distance between the X-ray tube and the X-ray detector by using a position of the X-ray tube that is acquired from the drive mechanism unit of each axis and a center position of the X-ray detector.

6. The X-ray imaging apparatus according to claim 1, wherein the processor is configured to:

use a maximum aperture of the X-ray collimator and an irradiation field of view at a reference value of the distance between the X-ray tube and the X-ray detector to obtain a relationship between the distance between the X-ray tube and the X-ray detector and an irradiation field size at the distance in advance; and
automatically control the aperture of the X-ray collimator in accordance with the size of the X-ray detector based on the relationship.

7. The X-ray imaging apparatus according to claim 1,

wherein the X-ray collimator includes a pair of left and right collimator blades and a pair of upper and lower collimator blades, and a mechanism that symmetrically opens and closes the pair of left and right collimator blades or the pair of upper and lower collimator blades with respect to a collimator center, and
the processor is configured to:

determine whether or not a line connecting a focal spot of the X-ray tube and a center position of the X-ray detector coincides with an irradiation direction of X-rays emitted from the X-ray tube; and
execute the aperture automatic control function only in a case where the line coincides with the irradiation direction.

8. The X-ray imaging apparatus according to claim 1, further comprising:

an operation panel connected to the processor and configured to interact with a user,
wherein the processor is configured to:

display a button (GUI) for the user to select the aperture automatic control function on the operation panel; and
execute the aperture automatic control function in a case where the button of the operation panel is operated.

9. The X-ray imaging apparatus according to claim 8, wherein the processor is configured to display at least one of the size of the X-ray detector or a size of an X-ray irradiation field achieved by the aperture automatic control function on the operation panel.

10. The X-ray imaging apparatus according to claim 8,

wherein the X-ray collimator includes a pair of left and right collimator blades and a pair of upper and lower collimator blades, and a mechanism that symmetrically opens and closes the pair of left and right collimator blades or the pair of upper and lower collimator blades with respect to a collimator center, and
the processor is configured to activate the button only in a case where a line connecting a focal spot of the X-ray tube and a center position of the X-ray detector coincides with an irradiation direction of X-rays emitted from the X-ray tube.

11. The X-ray imaging apparatus according to claim 1,

wherein the imaging table includes at least one of a table for imaging a subject in a decubitus position or an upright imaging stand for imaging the subject in an upright position, and
an axis for detecting the distance between the X-ray tube and the X-ray detector is changed between decubitus imaging and upright imaging.

12. An automatic control method for an X-ray collimator aperture of an X-ray imaging apparatus including an X-ray irradiation unit including an X-ray tube and an X-ray collimator, and an imaging table provided with an accommodating portion that accommodates an X-ray detector, the automatic control method comprising:

detecting a size of the X-ray detector accommodated in the accommodating portion and a distance between the X-ray tube and the X-ray detector;
calculating an X-ray collimator aperture corresponding to the size of the X-ray detector by using the detected size of the X-ray detector and the detected distance between the X-ray tube and the X-ray detector; and
automatically controlling the X-ray collimator to achieve the calculated X-ray collimator aperture.

**13.** The automatic control method for an X-ray collimator aperture according to claim 12,

wherein, in the calculation of the X-ray collimator aperture,
a maximum aperture of the X-ray collimator and an irradiation field of view at a reference value of the distance between the X-ray tube and the X-ray detector are used to calculate a relationship between the distance between the X-ray tube and the X-ray detector and an irradiation field size in advance, and
based on the relationship, an aperture is calculated such that the irradiation field size coincides with the size of the X-ray detector.

**14.** The automatic control method for an X-ray collimator aperture according to claim 13,
wherein the relationship between the distance between the X-ray tube and the X-ray detector and the irradiation field size is represented by a table or a function.

FIG. 1

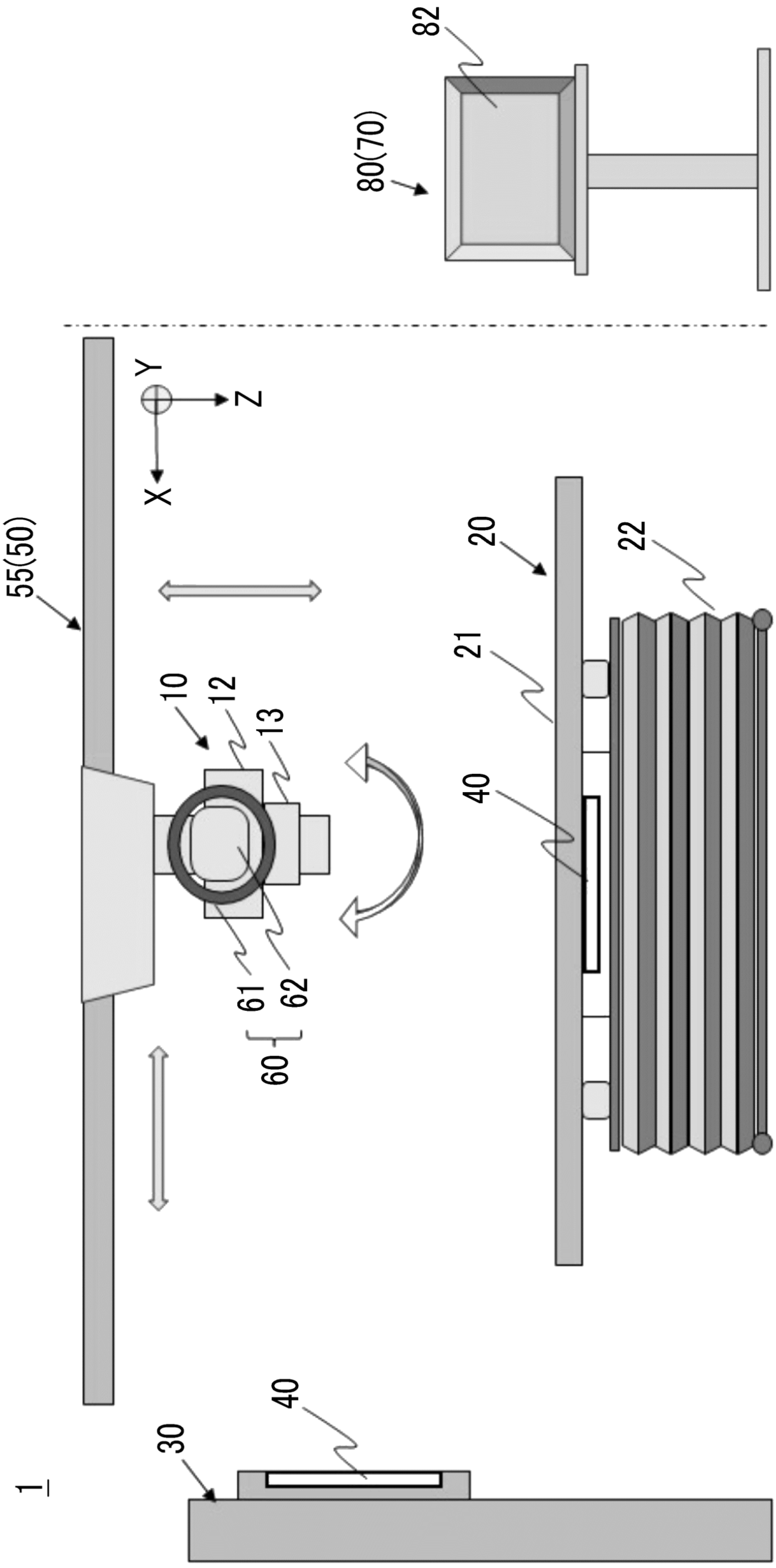

FIG. 2

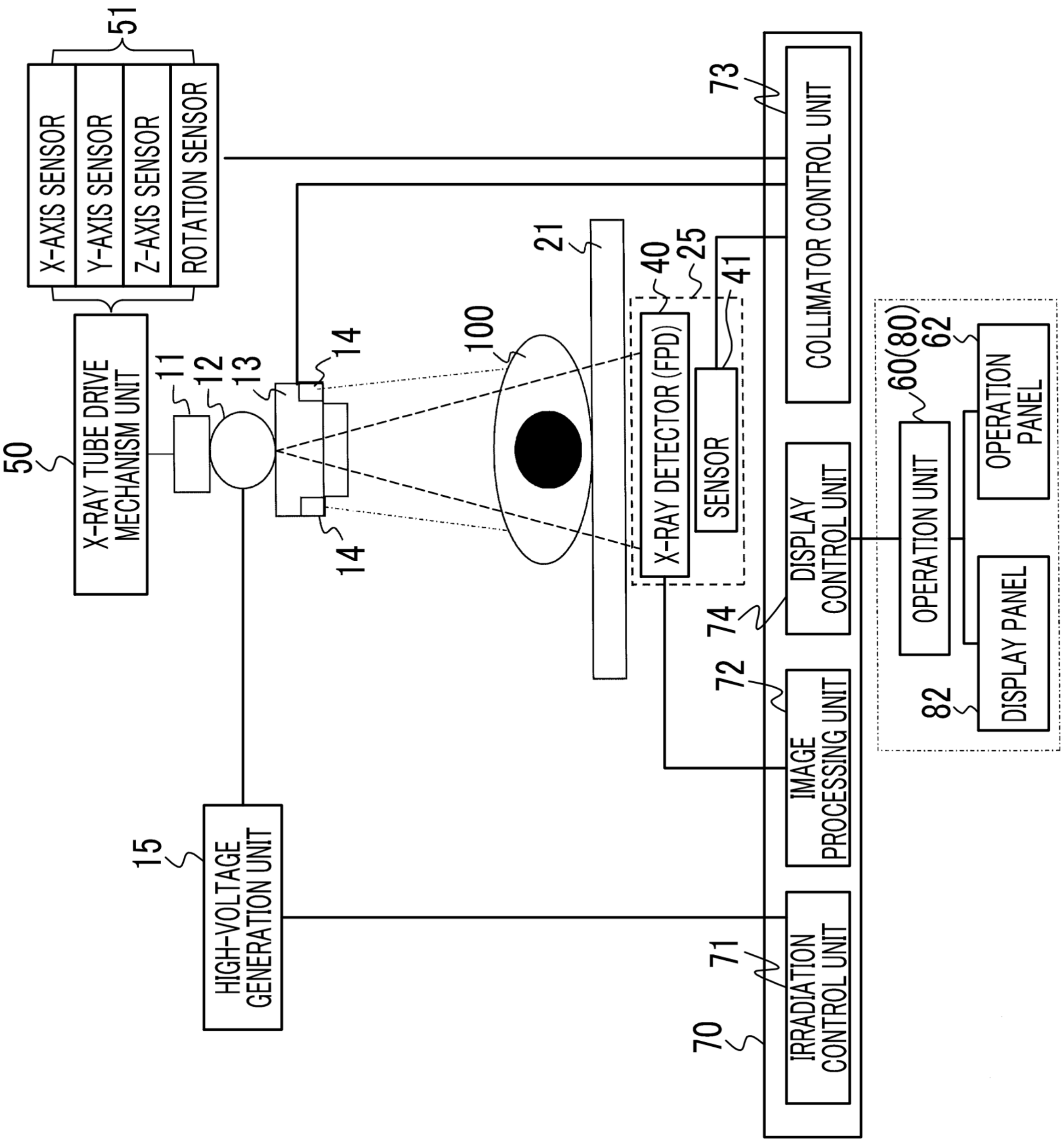
X-AXIS SENSOR
Y-AXIS SENSOR
Z-AXIS SENSOR
ROTATION SENSOR
51
50
X-RAY TUBE DRIVE MECHANISM UNIT
11
12
13
14
14
100
21
40
25
41
X-RAY DETECTOR (FPD)
SENSOR
73
COLLIMATOR CONTROL UNIT
15
HIGH-VOLTAGE GENERATION UNIT
70
71
IRRADIATION CONTROL UNIT
72
IMAGE PROCESSING UNIT
74
DISPLAY CONTROL UNIT
60(80)
OPERATION UNIT
62
OPERATION PANEL
82
DISPLAY PANEL

FIG. 3

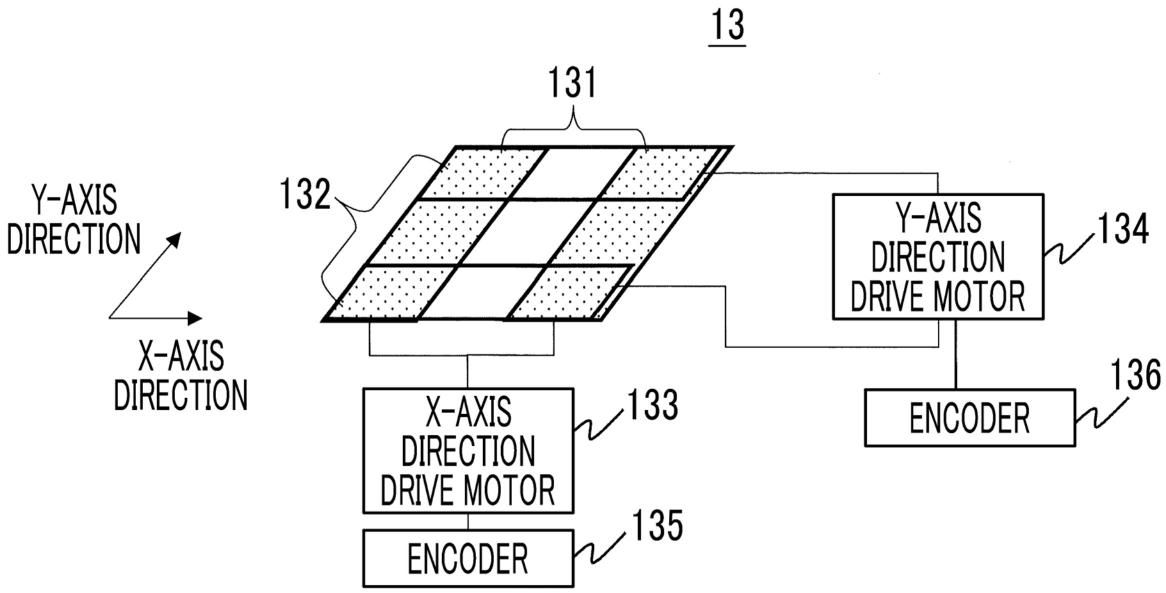
13
131
132
Y-AXIS DIRECTION
X-AXIS DIRECTION
Y-AXIS DIRECTION DRIVE MOTOR
134
X-AXIS DIRECTION DRIVE MOTOR
133
ENCODER
136
ENCODER
135

FIG. 4

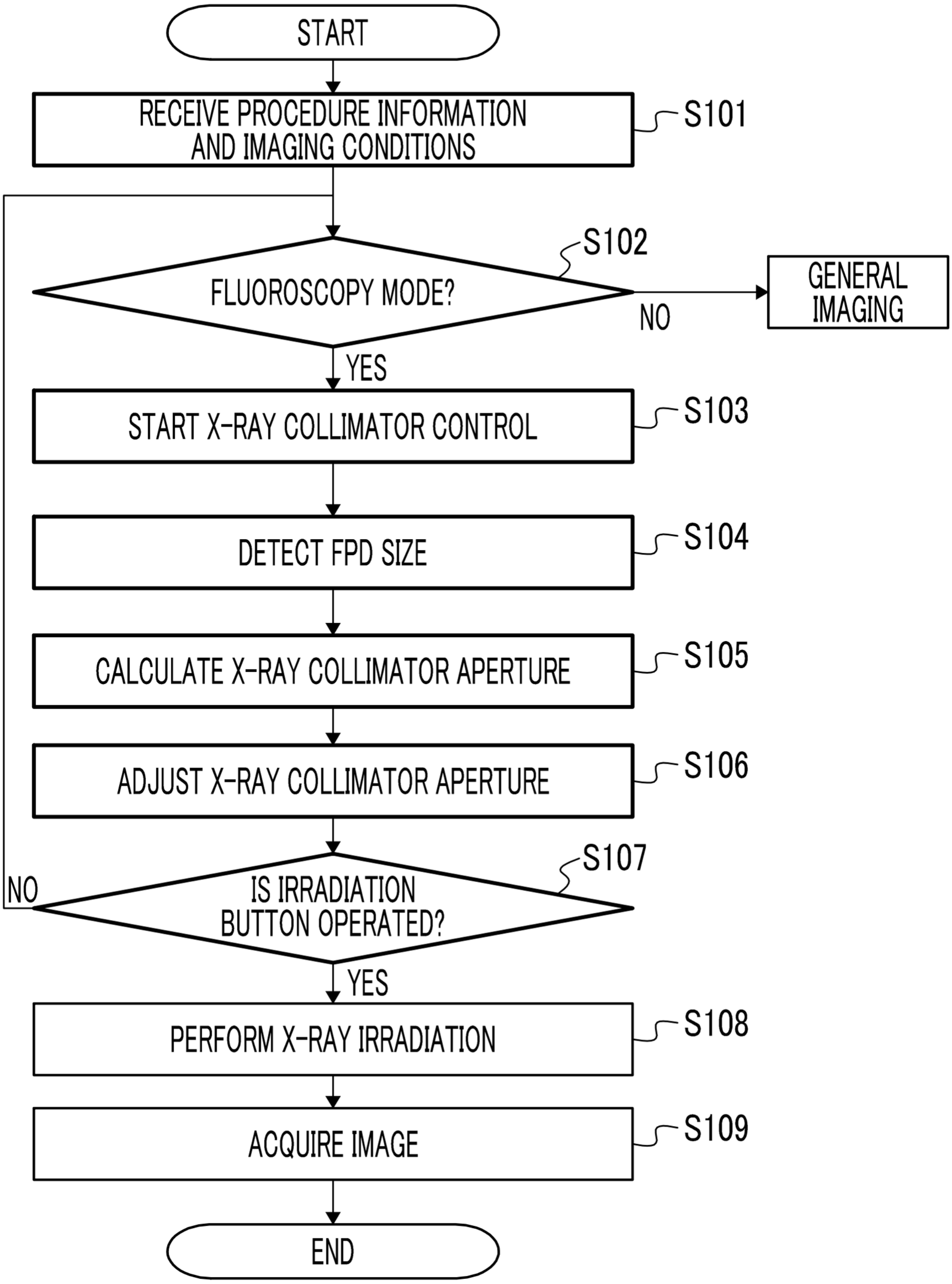
START
RECEIVE PROCEDURE INFORMATION AND IMAGING CONDITIONS
S101
FLUOROSCOPY MODE?
S102
NO
GENERAL IMAGING
YES
START X-RAY COLLIMATOR CONTROL
S103
DETECT FPD SIZE
S104
CALCULATE X-RAY COLLIMATOR APERTURE
S105
ADJUST X-RAY COLLIMATOR APERTURE
S106
IS IRRADIATION BUTTON OPERATED?
S107
NO
YES
PERFORM X-RAY IRRADIATION
S108
ACQUIRE IMAGE
S109
END

FIG. 5

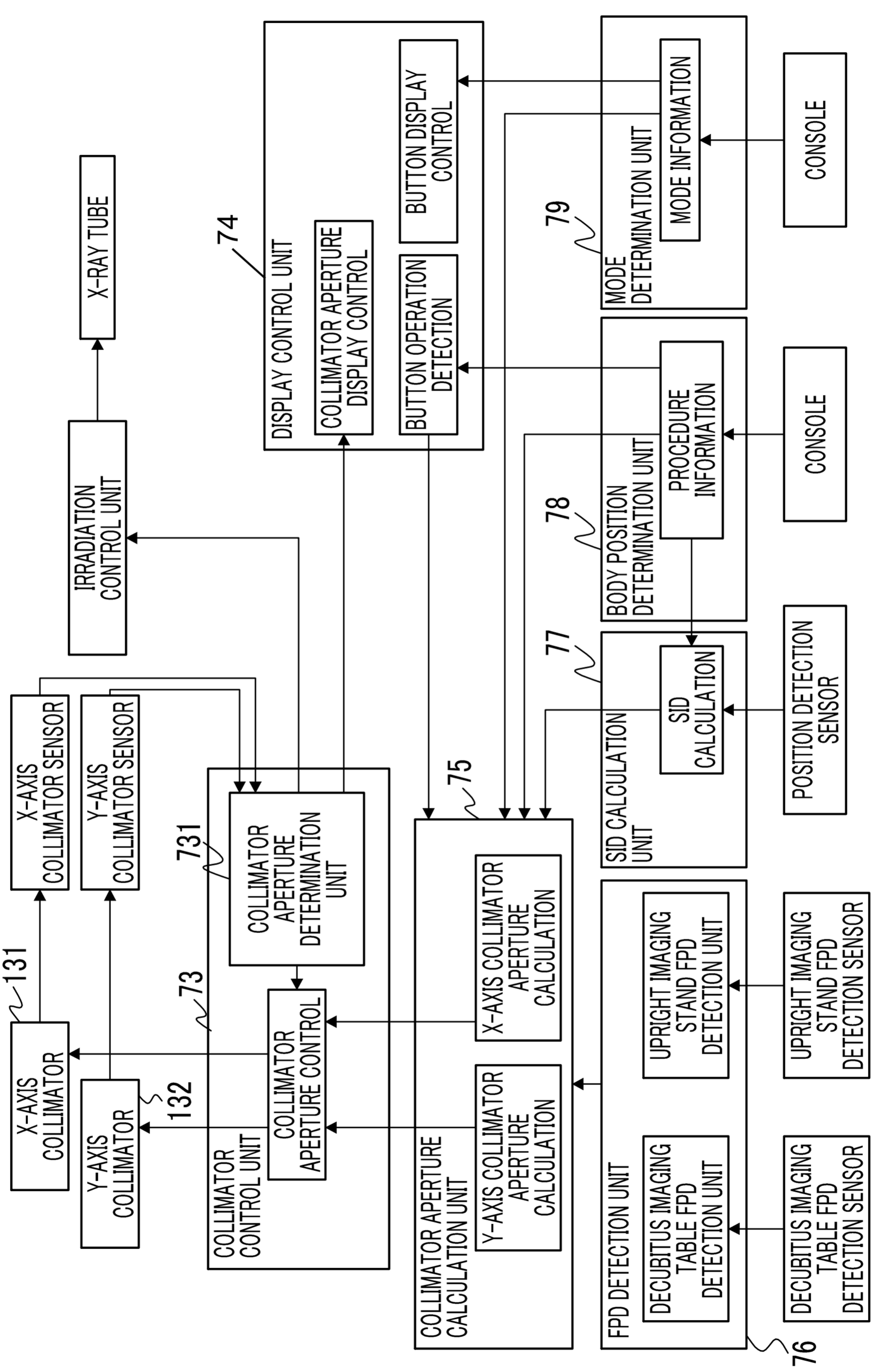
X-RAY TUBE
IRRADIATION CONTROL UNIT
X-AXIS COLLIMATOR SENSOR
Y-AXIS COLLIMATOR SENSOR
X-AXIS COLLIMATOR
Y-AXIS COLLIMATOR
131
132
73
731
COLLIMATOR CONTROL UNIT
COLLIMATOR APERTURE DETERMINATION UNIT
COLLIMATOR APERTURE CONTROL
74
DISPLAY CONTROL UNIT
COLLIMATOR APERTURE DISPLAY CONTROL
BUTTON OPERATION DETECTION
BUTTON DISPLAY CONTROL
75
COLLIMATOR APERTURE CALCULATION UNIT
X-AXIS COLLIMATOR APERTURE CALCULATION
Y-AXIS COLLIMATOR APERTURE CALCULATION
79
MODE DETERMINATION UNIT
MODE INFORMATION
CONSOLE
78
BODY POSITION DETERMINATION UNIT
PROCEDURE INFORMATION
CONSOLE
77
SID CALCULATION UNIT
SID CALCULATION
POSITION DETECTION SENSOR
76
FPD DETECTION UNIT
UPRIGHT IMAGING STAND FPD DETECTION UNIT
DECUBITUS IMAGING TABLE FPD DETECTION UNIT
UPRIGHT IMAGING STAND FPD DETECTION SENSOR
DECUBITUS IMAGING TABLE FPD DETECTION SENSOR

FIG. 6

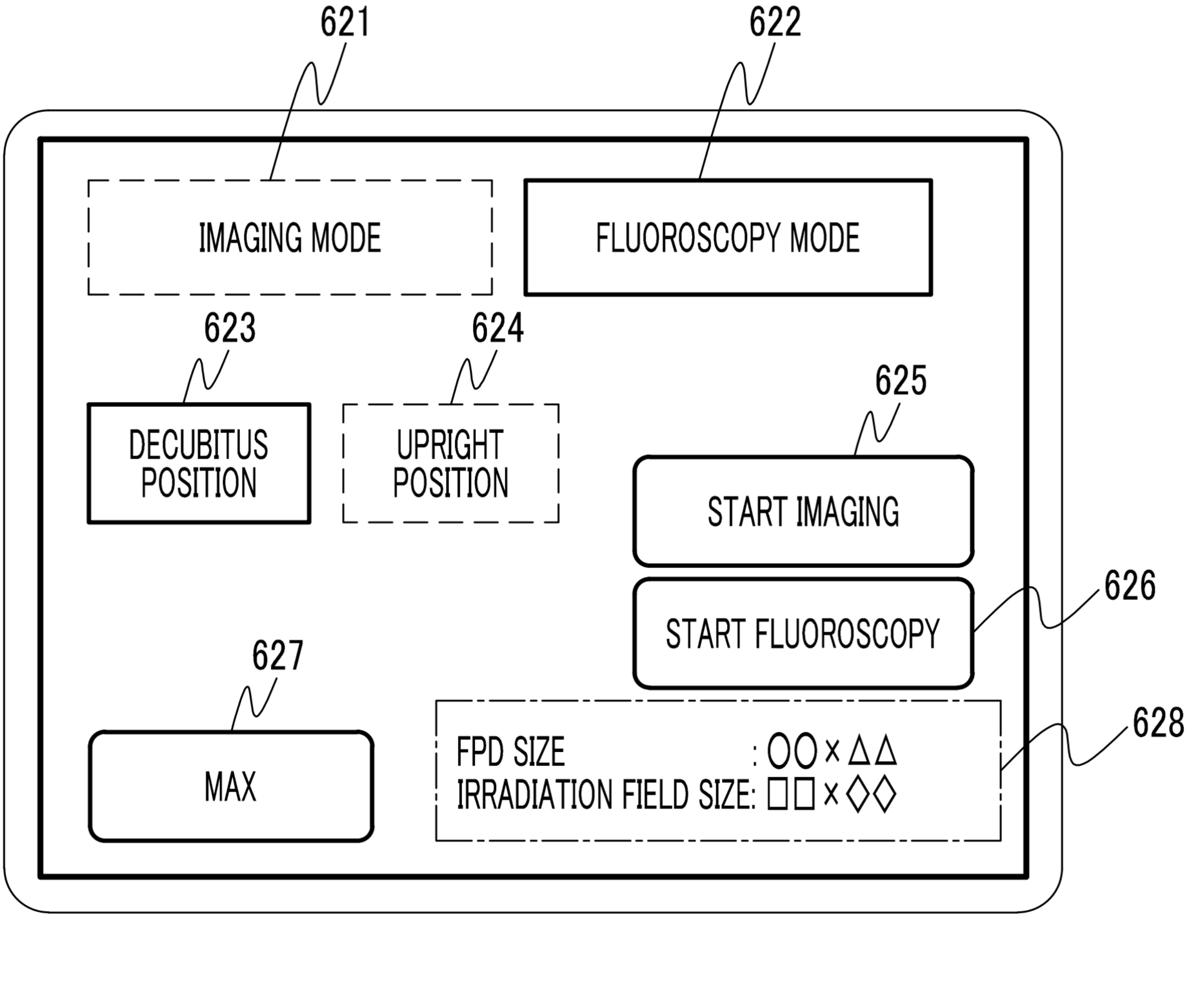
621
622
IMAGING MODE
FLUOROSCOPY MODE
623
624
DECUBITUS POSITION
UPRIGHT POSITION
625
START IMAGING
START FLUOROSCOPY
626
627
MAX
628
FPD SIZE : ○○×△△
IRRADIATION FIELD SIZE: □□×◇◇

# FIG. 7

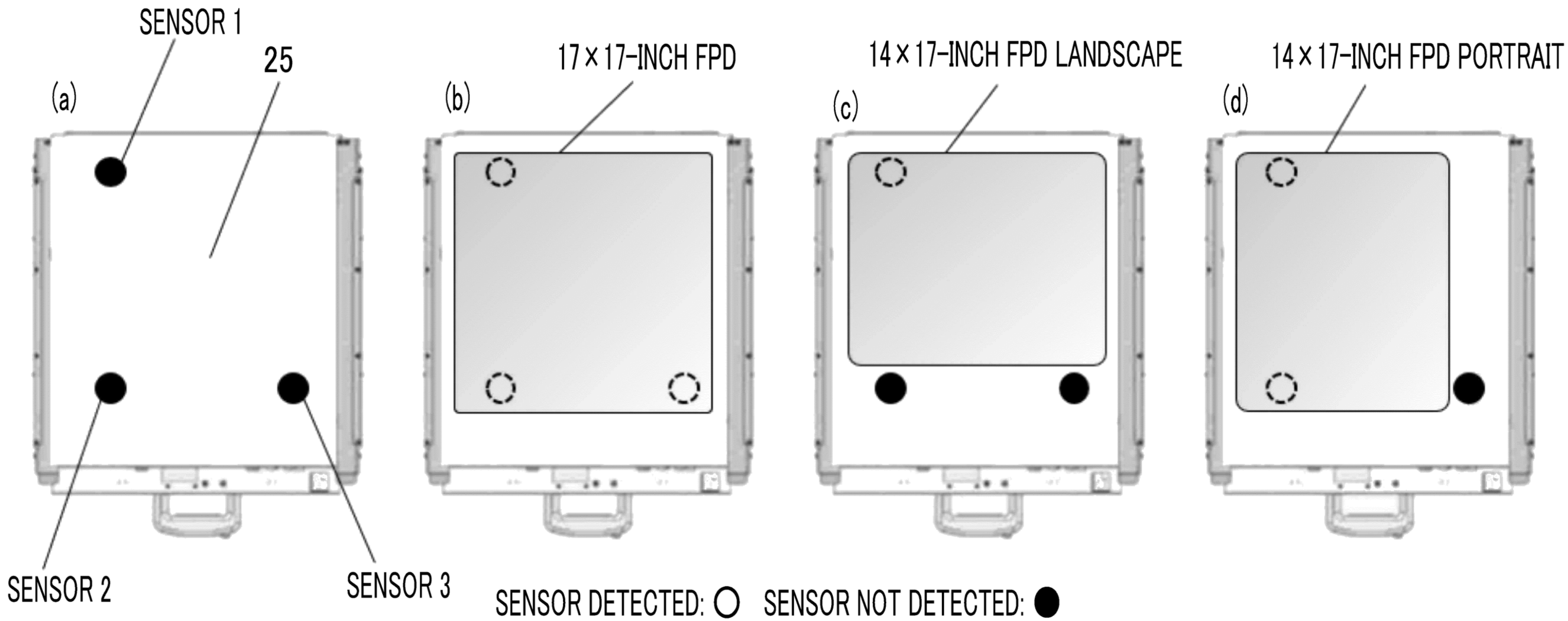


SENSOR DETECTED: ○ SENSOR NOT DETECTED: ●

| | (a) NO FPD | (b) 17×17-INCH FPD | (c) 14×17-INCH FPD LANDSCAPE | (d) 14×17-INCH FPD PORTRAIT |
|---|---|---|---|---|
| SENSOR 1 | ● | ○ | ○ | ○ |
| SENSOR 2 | ● | ○ | ● | ○ |
| SENSOR 3 | ● | ○ | ● | ● |

FIG. 8

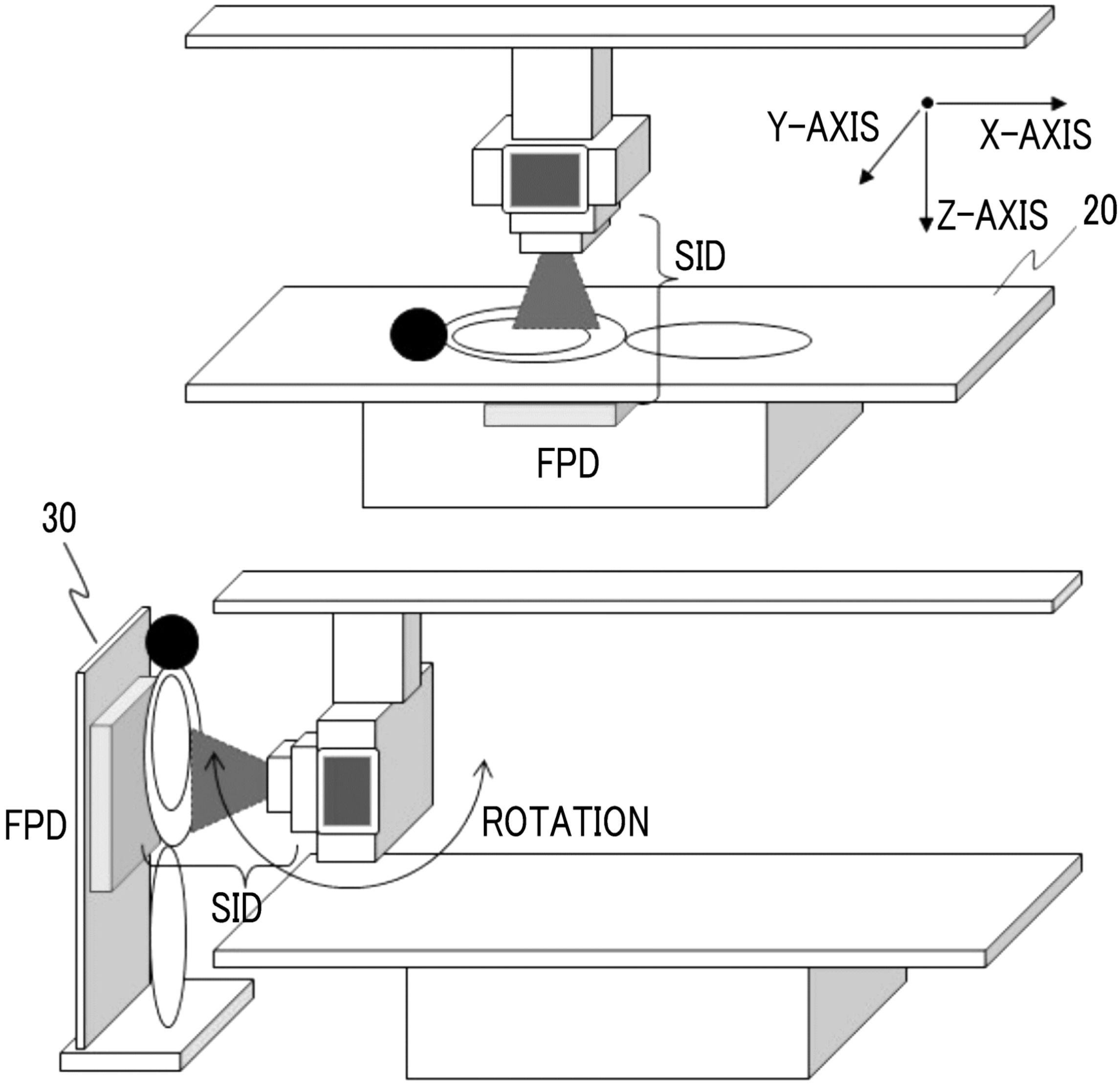

FIG. 9

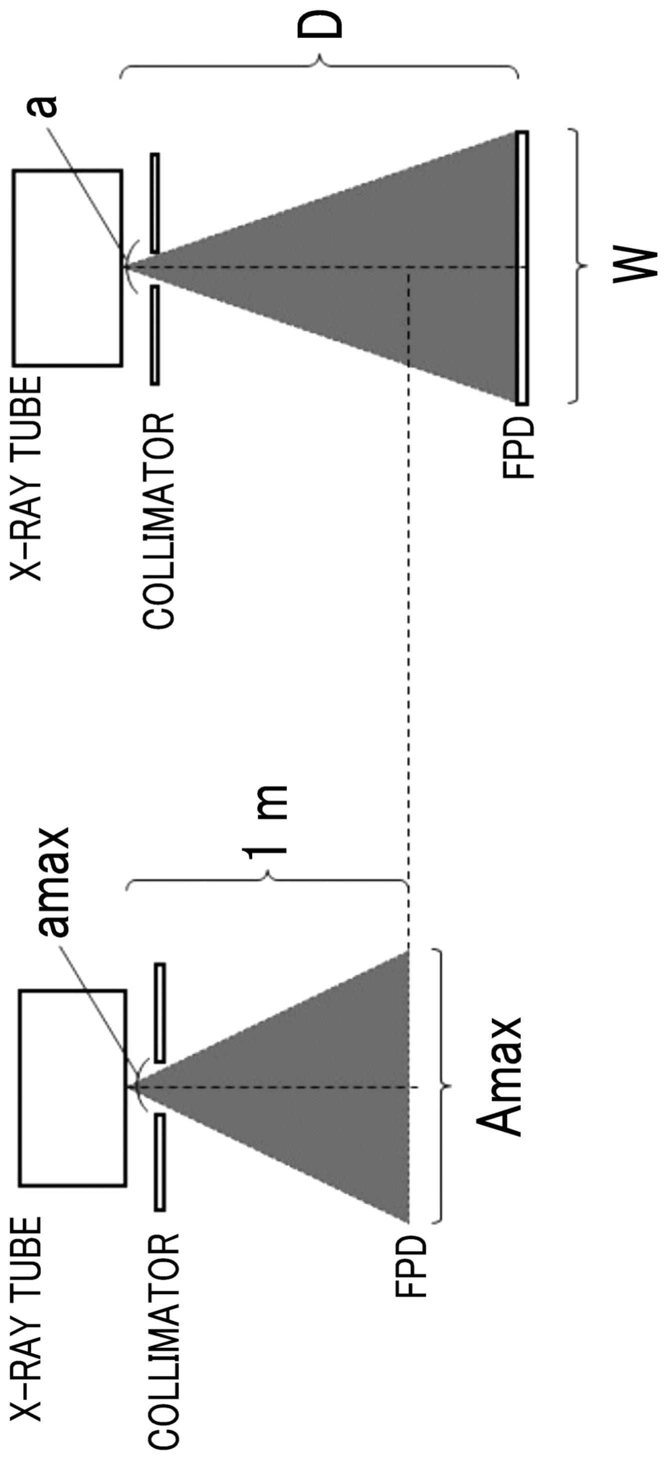
X-RAY TUBE
COLLIMATOR
FPD
a
D
W
X-RAY TUBE
COLLIMATOR
FPD
amax
1 m
Amax

FIG. 10A

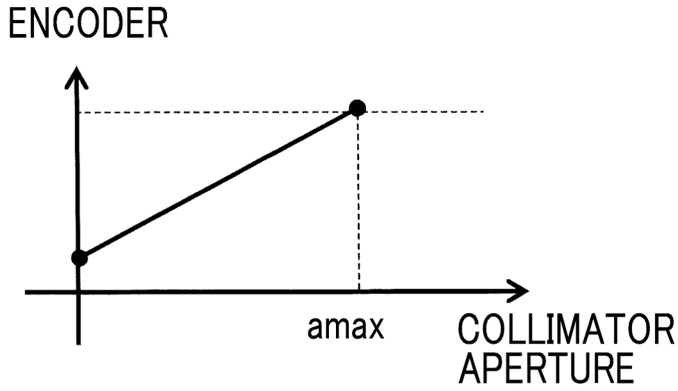


FIG. 10B

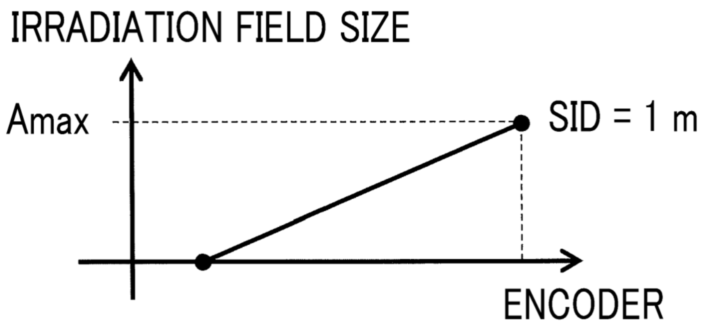


FIG. 10C

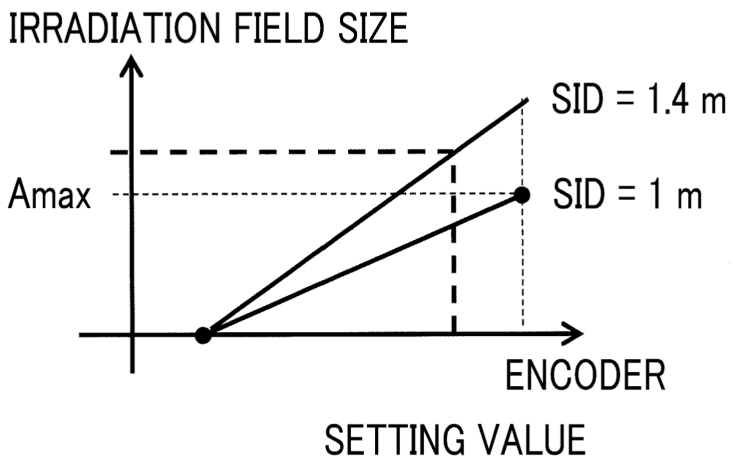

FIG. 11

TABLE (EXAMPLE) FOR DETERMINING COLLIMATOR APERTURE FROM SID AND IRRADIATION FIELD SIZE

| | | SID = 1 m | SID = 1.5 m | SID = 2.0 m |
|---|---|---|---|---|
| 50 | 0 | 0 | 0 | 0 |
| : | : | : | : | : |
| : | : | : | : | : |
| 153 | 6.2 cm | 49 cm | 58 cm | 69 cm |
| ENCODER | COLLIMATOR APERTURE | | | |

← COLLIMATOR FULLY CLOSED

← COLLIMATOR FULLY OPEN

IRRADIATION FIELD SIZE

FIG. 12

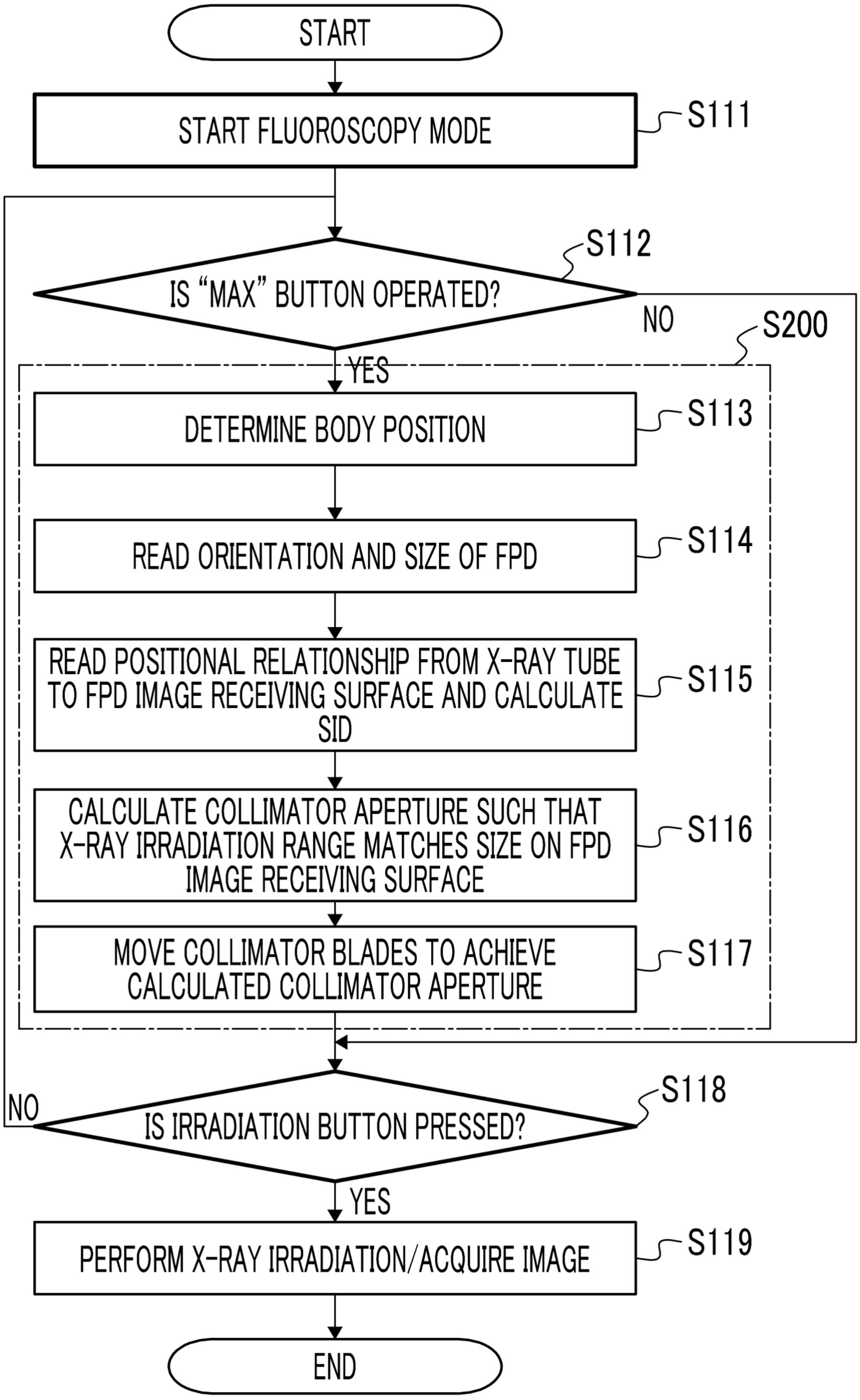
START
START FLUOROSCOPY MODE
S111
S112
IS "MAX" BUTTON OPERATED?
NO
S200
YES
DETERMINE BODY POSITION
S113
READ ORIENTATION AND SIZE OF FPD
S114
READ POSITIONAL RELATIONSHIP FROM X-RAY TUBE TO FPD IMAGE RECEIVING SURFACE AND CALCULATE SID
S115
CALCULATE COLLIMATOR APERTURE SUCH THAT X-RAY IRRADIATION RANGE MATCHES SIZE ON FPD IMAGE RECEIVING SURFACE
S116
MOVE COLLIMATOR BLADES TO ACHIEVE CALCULATED COLLIMATOR APERTURE
S117
S118
NO
IS IRRADIATION BUTTON PRESSED?
YES
PERFORM X-RAY IRRADIATION/ACQUIRE IMAGE
S119
END

FIG. 13A

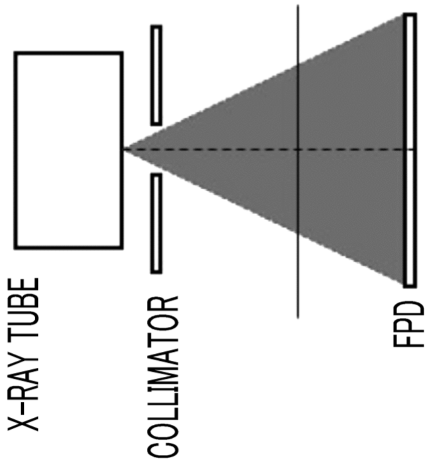


FIG. 13B

CHANGE POSITIONAL RELATIONSHIP FROM X-RAY TUBE TO FPD IMAGE RECEIVING SURFACE (IN LATERAL DIRECTION)

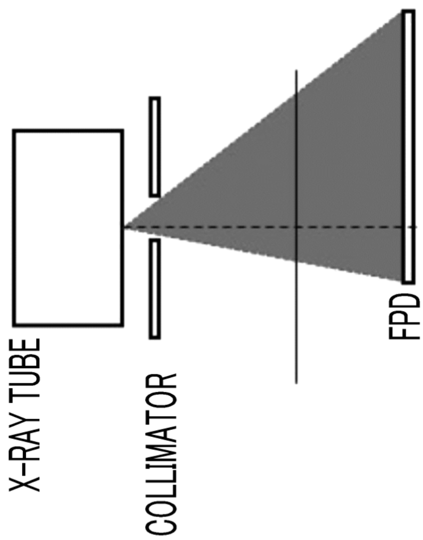


FIG. 13C

CHANGE POSITIONAL RELATIONSHIP FROM X-RAY TUBE TO FPD IMAGE RECEIVING SURFACE (IN CASE OF OBLIQUE INCIDENCE)

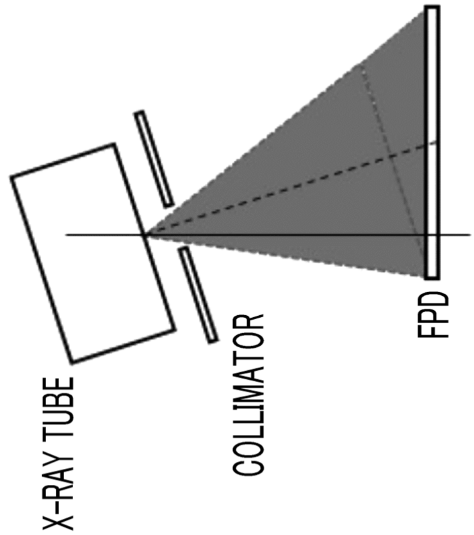

FIG. 14

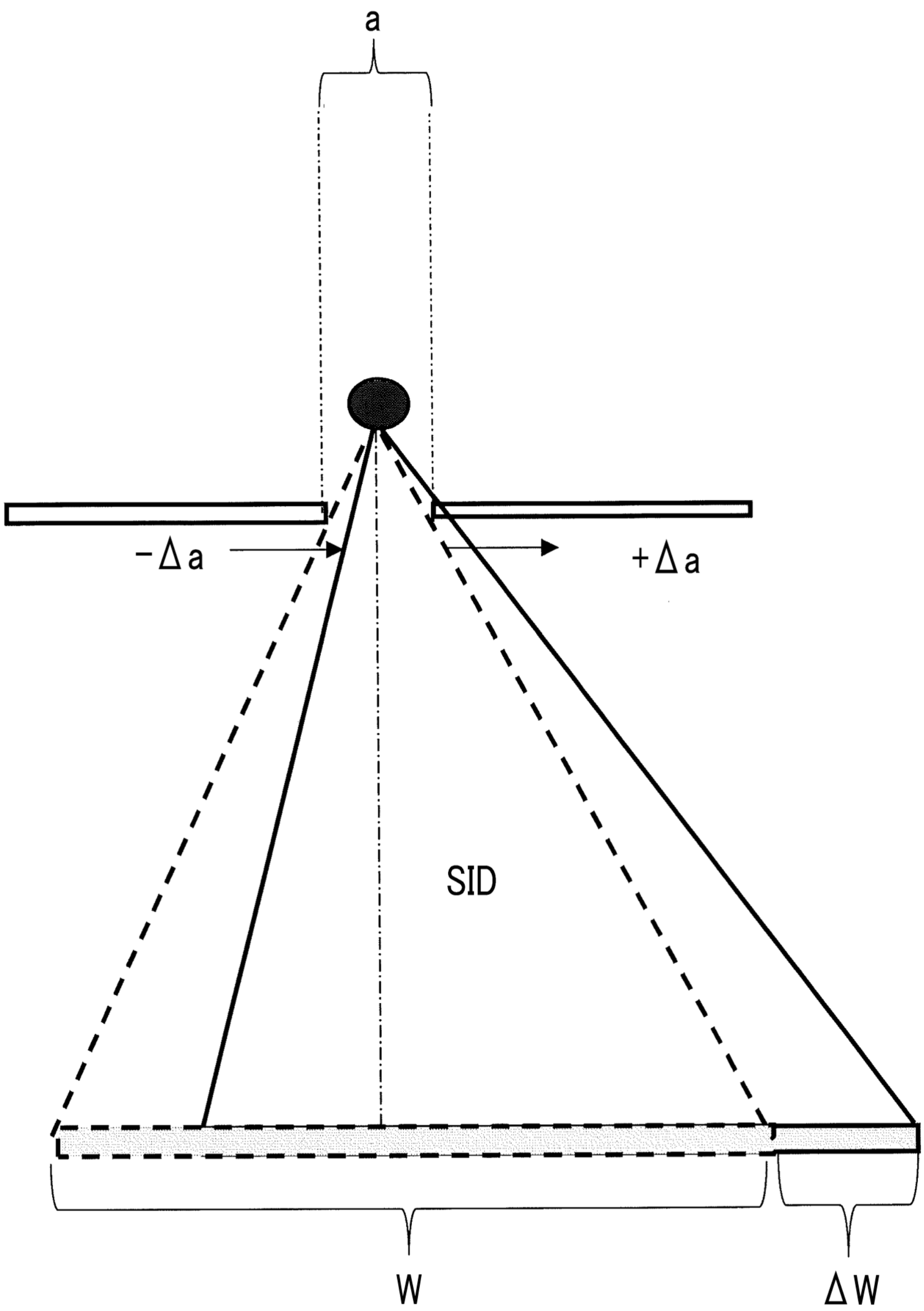

Europäisches Patentamt
European Patent Office
Office européen des brevets

# EUROPEAN SEARCH REPORT

Application Number
EP 26 15 2182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | US 2019/290236 A1 (OEPPING SUSANNE [DE] ET AL) 26 September 2019 (2019-09-26)<br>* paragraph [0061] - paragraph [0090]; figure 1 *<br>----- | 1-3,5-14<br>4 | INV.<br>A61B6/06<br>A61B6/00<br>A61B6/58 |
| Y<br>A | WO 96/37088 A1 (CONTINENTAL X RAY CORP [US]) 21 November 1996 (1996-11-21)<br>* page 8, line 34 - page 33, line 10; figures 1, 6, 9 *<br>----- | 1-3,5-14<br>4 | ADD.<br>A61B6/04<br>A61B6/42 |
| A | WO 2014/125090 A1 (KONINKL PHILIPS NV [NL]; PHILIPS DEUTSCHLAND GMBH [DE]) 21 August 2014 (2014-08-21)<br>* page 5, line 26 - page 21, line 29 *<br>----- | 1-14 | |
| A | WO 2023/015110 A1 (OXOS MEDICAL INC [US]) 9 February 2023 (2023-02-09)<br>* paragraph [0071] - paragraph [0250] *<br>----- | 1-14 | |
| | | | TECHNICAL FIELDS SEARCHED (IPC) |
| | | | A61B |

The present search report has been drawn up for all claims

2

| Place of search | Date of completion of the search | Examiner |
|---|---|---|
| Munich | 29 May 2026 | Hooper, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 26 15 2182

This annex lists the patent family members relating to the patent documents cited in the above-mentioned European search report. The members are as contained in the European Patent Office EDP file on
The European Patent Office is in no way liable for these particulars which are merely given for the purpose of information.

29-05-2026

| Patent document cited in search report | Publication date | Patent family member(s) | Publication date |
|---|---|---|---|
| US 2019290236 A1 | 26-09-2019 | CN 110353721 A | 22-10-2019 |
| | | EP 3545846 A1 | 02-10-2019 |
| | | US 2019290236 A1 | 26-09-2019 |
| WO 9637088 A1 | 21-11-1996 | CA 2215703 A1 | 21-11-1996 |
| | | EP 0873672 A1 | 28-10-1998 |
| | | US 5636259 A | 03-06-1997 |
| | | US 5751788 A | 12-05-1998 |
| | | US 5768336 A | 16-06-1998 |
| | | WO 9637088 A1 | 21-11-1996 |
| WO 2014125090 A1 | 21-08-2014 | CN 104994788 A | 21-10-2015 |
| | | EP 2767236 A1 | 20-08-2014 |
| | | EP 2956063 A1 | 23-12-2015 |
| | | JP 6456848 B2 | 23-01-2019 |
| | | JP 2016507306 A | 10-03-2016 |
| | | US 2015374314 A1 | 31-12-2015 |
| | | WO 2014125090 A1 | 21-08-2014 |
| WO 2023015110 A1 | 09-02-2023 | EP 4362804 A1 | 08-05-2024 |
| | | JP 2024529987 A | 14-08-2024 |
| | | US 11382582 B1 | 12-07-2022 |
| | | US 2023039343 A1 | 09-02-2023 |
| | | US 2023040643 A1 | 09-02-2023 |
| | | US 2023270395 A1 | 31-08-2023 |
| | | US 2024065656 A1 | 29-02-2024 |
| | | US 2024206831 A1 | 27-06-2024 |
| | | US 2025072851 A1 | 06-03-2025 |
| | | US 2025255562 A1 | 14-08-2025 |
| | | WO 2023015110 A1 | 09-02-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2025006471 A
- JP 2005031323 A
- JP 2015026313 A
- JP 2008036314 A
- JP 2008 A
- JP 36314 A